# EUROPEAN PATENT SPECIFICATION

(11) **EP 3 592 155 B1**
(45) Date of publication and mention of the grant of the patent: **06.05.2026**
(21) Application number: 18708139.3
(22) Date of filing: 07.03.2018
(51) Int. Cl.: A61K 38/47, A23B 4/22, C12N 9/36

(54) **METHOD FOR REDUCING CONTAMINATION OF AN OBJECT WITH CLOSTRIDIUM**
VERFAHREN ZUR VERRINGERUNG DER VERSCHMUTZUNG EINES OBJEKTS MIT CLOSTRIDIUM
PROCÉDÉ DE RÉDUCTION DE LA CONTAMINATION D'UN OBJET PAR CLOSTRIDIUM

(30) Priority: 07.03.2017 EP 17159597
(43) Date of publication of application: 15.01.2020
(73) Proprietor: Nomad Bioscience GmbH, 80333 München (DE)
(72) Inventor: KAZANAVICIUTE, Vaiva, 08250 Vilnius (LT); MISIUNAS, Audrius, 06152 Vilnius (LT); RAZANSKIENE, Ausra, 08240 Vilnius (LT)
(74) Representative: Wächtershäuser & Hartz Patentanwaltspartnerschaft mbB
(86) International application number: PCT/EP2018/055635
(87) International publication number: WO 2018/162570

(56) References cited:
- EP-A1- 2 143 729
- WO-A1-2010/136754
- WO-A1-2010/136754
- US-A1- 2012 052 546
- US-A1- 2012 052 546
- DATABASE Geneseq [online] 1 April 2010 (2010-04-01), "Lysin plyS9 protein sequence, SEQ ID 1.", retrieved from EBI accession no. GSP:AXV31492 Database accession no. AXV31492
- DATABASE Geneseq [online] 1 April 2010 (2010-04-01), "Lysin plyS9 protein C-terminal fragment sequence, SEQ ID 13.", retrieved from EBI accession no. GSP:AXV31504 Database accession no. AXV31504
- STARKEVIC URTE ET AL: "High-yield production of a functional bacteriophage lysin with antipneumococcal activity using a plant virus-based expression system", JOURNAL OF BIOTECHNOLOGY, ELSEVIER, AMSTERDAM NL, vol. 200, 2 March 2015 (2015-03-02), pages 10 - 16, XP029215393, ISSN: 0168-1656, DOI: 10.1016/J.JBIOTEC.2015.02.028
- TERESA GERVASI ET AL: "Expression and delivery of an endolysin to combat Clostridium perfringens", APPLIED MICROBIOLOGY AND BIOTECHNOLOGY, vol. 98, no. 6, 15 August 2013 (2013-08-15), pages 2495 - 2505, XP055117881, ISSN: 0175-7598, DOI: 10.1007/s00253-013-5128-y
- BETTY C HOBBS ET AL: "CLOSTRIDIUM WELCHII FOOD POISONING", J HYG (LOND)., vol. 51, no. 1, 1 March 1953 (1953-03-01), London, XP055391993, Retrieved from the Internet <URL:https://www.ncbi.nlm.nih.gov/pmc/articles/PMC2217678/pdf/jhyg00159-0089.pdf> [retrieved on 20170718]
- TERESA GERVASI ET AL: "Expression and delivery of an endolysin to combat Clostridium perfringens", APPLIED MICROBIOLOGY AND BIOTECHNOLOGY, vol. 98, no. 6, 15 August 2013 (2013-08-15), pages 2495 - 2505, XP055117881, ISSN: 0175-7598, DOI: 10.1007/s00253-013-5128-y
- BETTY C HOBBS ET AL: "CLOSTRIDIUM WELCHII FOOD POISONING", J HYG (LOND)., vol. 51, no. 1, 1 March 1953 (1953-03-01), London, XP055391993, Retrieved from the Internet <URL:https://www.ncbi.nlm.nih.gov/pmc/articles/PMC2217678/pdf/jhyg00159-0089.pdf> [retrieved on 20170718]

## Description

### FIELD OF THE INVENTION

The present invention relates to method of preventing or reducing contamination of an object such as food or animal feed with *Clostridium.* The invention further relates to a process of producing a composition comprising at least one endolysin active against *Clostridium,* said process comprising expressing said endolysin in a plant or in plant cells. The invention further relates to a composition comprising an endolysin active against *Clostridium,* and to a method of treating an infection with *Clostridium* such as *Clostridium difficile* or *Clostridium perfringens.*

### BACKGROUND OF THE INVENTION

The anaerobic spore-forming bacterium *Clostridium perfringens* is a source of one of the most common food-borne illnesses in the United States and Europe. C. *perfringens* gastroenteritis is caused by type A strains (producing alphatoxin encoded by the cpa gene, also known as phospholipase C, encoded by the plc gene) that produce the C. *perfringens* enterotoxin (CPE) (Xiao et al., 2012).

The CPE-mediated food poisoning outbreaks typically involve a large number of victims and are associated with temperature-abused meat or poultry dishes. Optimal conditions for food poisonings arise when food contaminated with CPE-positive C. *perfringens* spores is slowly chilled or held or served at a temperature range of 10-54 °C, allowing germination and rapid growth of C. *perfringens* (Li and McClane, 2006, Lindström et al., 2011). The costs associated with the disease are high. In the Netherlands, the average incidence of *Clostridium perfringens* gastroenteritis is estimated to be 171,000 cases per year, with total cost from food-related C. *perfringens* infection cases amounting to 23.2 million euros (Mangen et al., 2015).

In this regard, effective and safe antimicrobials to control food contamination by C. *perfringens* are needed. Currently, there are very few interventions targeted toward the inactivation of bacteria on food. Most of the available interventions involve heating or organic acids, which can adversely modify the taste and quality of the products. Recently, Schulz et al. (2015) described potential application of plant-produced colicins to control enterohaemoragic E. *coli* in food. EP 2 143 729 A1 also discloses an endolysin for the treatment, prevention or diagnosis of Clostridium contamination of foodstuff, of food processing equipment, of food processing plants, or of surfaces coming into contact with foodstuff.

Bacteriophages, natural enemies of bacteria, have been used during the early part of the 20^{th} century, mostly as therapeutic agents before the discovery of antibiotics. The growing problem of antibiotic resistant pathogens renewed interest in bacteriophage therapy and applications in other fields, where bacterial contamination is of concern, including food treatment. Phage cocktails (Listex P100, SalmoFresh, ListShield) developed by Intralytix have been commercialized. The same company also developed and licensed out the phage product INT-401 ^{™} for veterinary application, for controlling of *Clostridium perfringens* in poultry (Miller et al., 2010).

The inventors considered that bacteriophage lysins may be even safer alternatives compared to whole bacteriophages and conceived that bacteriophage lysins used as food additives or used for food processing could be a viable solution for controlling *Clostridium perfringens* and other Clostridia. Antimicrobial activity of several E. coli-produced C. *perfringens* lysins such as Ply3626, PlyCP26F, PlyCP39O, psm, PlyCM and CP25L has been described (Zimmer et al. 2002; Simmons et al., 2010; Nariya et al., 2011; Schmitz et al., 2011; Gervasi et al., 2014).

Departing from the prior art, it is an object of the invention to provide a method and an agent for preventing or reducing contamination of an object such as food or animal feed with *Clostridium,* notably with *Clostridium perfringens.* It is another object to provide a process of producing a composition comprising at least one endolysin active against *Clostridium.* It is a further object to provide a composition active against *Clostridium,* and a method of treating an infection with *Clostridium* such as *Clostridium difficile* or *Clostridium perfringens.*

### SUMMARY OF THE INVENTION

The present invention provides the following:
(1) A method of preventing or reducing contamination of food with *Clostridium,* comprising contacting said food with a composition comprising at least one endolysin active against said *Clostridium,*
   wherein at least one of said at least one endolysin is ZP173 comprising the amino acid sequence of SEQ ID NO: 2 or a derivative of this endolysin, and
   wherein said derivative or endolysin has a sequence identity of at least 90% to the amino acid sequence of SEQ ID NO: 2; or said derivative or endolysin has from 1 to 30 amino acid substitutions, insertions and/or deletions compared to the amino acid sequence of SEQ ID NO: 2.
(2) The method according to item 1, wherein said composition is or comprises a plant material or extract thereof, wherein the plant material is a material from a plant having expressed said at least one endolysin, preferably an edible plant having expressed said at least one endolysin.
(3) The method according to item 1 or 2, wherein said composition is an aqueous solution containing said at least one endolysin.
(4) The method according to item 3, wherein said aqueous solution containing said at least one endolysin is a buffered aqueous solution that contains from 50 to 400 mM NaCl, preferably from 140 to 310 mM, and may further contain a sulfhydryl compound.
(5) The method according to any one of items 1 to 4, wherein said food is sprayed with an aqueous solution containing said at least one endolysin or is immersed into an aqueous solution containing said at least one endolysin.
(6) The method according to any one of items 1 to 5, wherein said food is meat, preferably said meat is raw meat or cooked meat; or said food is gravy.
(7) The method according to any one of items 1 to 6, wherein said *Clostridium* is *Clostridium perfringens,* preferably anyone or more selected from of Type A, Type B, Type C, Type D and Type **E,** preferably a Type A strain containing enterotoxin CPE.
(8) The method according to item 1, wherein said derivative or endolysin has a sequence identity of at least 95%, preferably at least 97% to an amino acid sequence of SEQ ID NO: 2; or
   said derivative has from 1 to 15, preferably from 1 to 10, amino acid substitutions, insertions and/or deletions compared to an amino acid sequence of SEQ ID NO: 2.
(9) The method according to item 1, wherein said composition is a buffered aqueous solution that contains from 150 to 700 mM NaCl, preferably from 200 to 550 mM NaCl, and/or the composition is an aqueous solution of a pH of from pH 4 to 8, preferably from pH 4.5 to 7, more preferably from 5.0 to 6.5, and even more preferably from 5.0 to 6.0.
(10) The method according to item 2, wherein said plant material is material from a plant selected from the group consisting of spinach, chard, beetroot, carrot, sugar beet, *Nicotiana tabacum, Nicotiana benthamiana* and/or said plant material is one or more leaves, roots, tubers, or seeds, or a crushed, milled or comminuted product of said leaves, roots, tubers, or seeds.
(11) A process of producing a composition comprising at least one endolysin active against *Clostridium,* said process comprising the following steps:
   (i) expressing said at least one endolysin in a plant, preferably an edible plant,
   (ii) harvesting plant material containing expressed endolysin from said plant,
   (iii) extracting said at least one endolysin from said plant material using an aqueous buffer to obtain a composition containing said at least one endolysin,
   (iv) optionally removing undesired contaminants from said composition;
      as further defined by claim 11.
(12) The process according to item 11, wherein step (iv) comprises, in this order, a hydrophobic interaction chromatography step, a desalting step, and an ion exchange chromatography step.
(13) A composition comprising an endolysin active against *Clostridium,* as defined by claim 13.
(14) The composition according to item 13 for use in a method of treating or preventing infection with *Clostridium* such as *Clostridium difficile* or *Clostridium perfringens.*

The inventors have identified lysins that have a broad bacteriolytic activity over a wide range of C. *perfringens* strains. The inventors have identified specific lysins and compositions that are particularly suited for preventing contamination of food with *Clostridium* due to particularly high stability over time, activity as well pH-dependence. Notably, lysins and compositions have been identified that are particularly suited for preventing contamination of meat with *Clostridium.* The inventors have further found that plant virus-based expression systems, notably when the foreign mRNA is amplified by a replicating virus, can produce very high levels of lysin active against *Clostridium* in leaves and other tissues. Plant-based production systems have clear advantages for producing proteins for which extensive purification may be avoided when these proteins are produced by GRAS organisms. Bacteriophage lysins targeting food pathogens and produced in edible plants can be safely used as food additives or processing aids even if only partially pure.

The present invention provides the first plant expression of C. *perfringens* lysins. It demonstrates not only successful expression in plants of bacteriophage lysins belonging to different families (N-acetylmuramoyl-L-alanine amidase and glycosyl hydrolase 25), but also the possibility to use plant-expressed lysins in semi-purified form, as crude protein extracts containing psm, ZP173 and ZP278, CP25L, PlyCP26F or PlyCP39O or derivatives thereof, and demonstrates lytic activity against tested C. *perfringens* strains *in vitro* and in food, such as in cooked meat.

### DESCRIPTION OF THE FIGURES

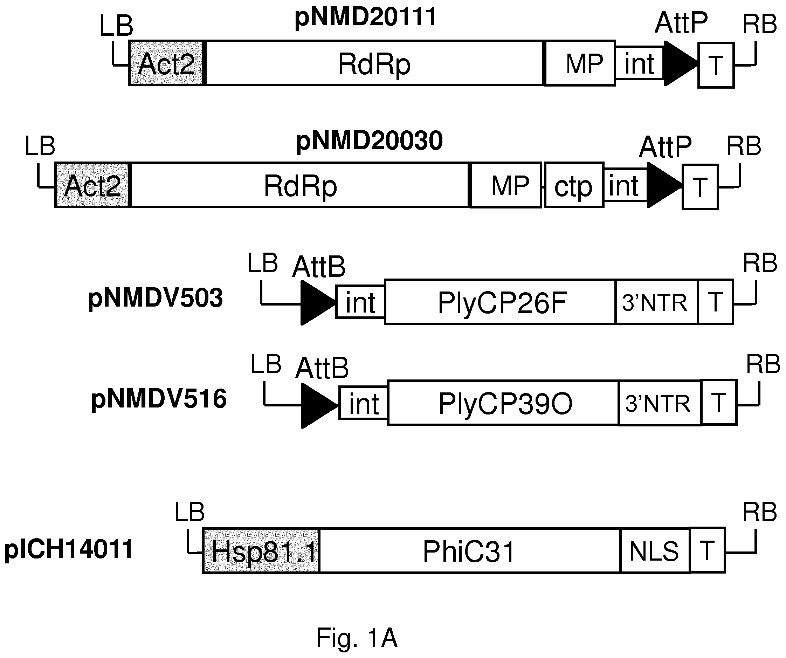

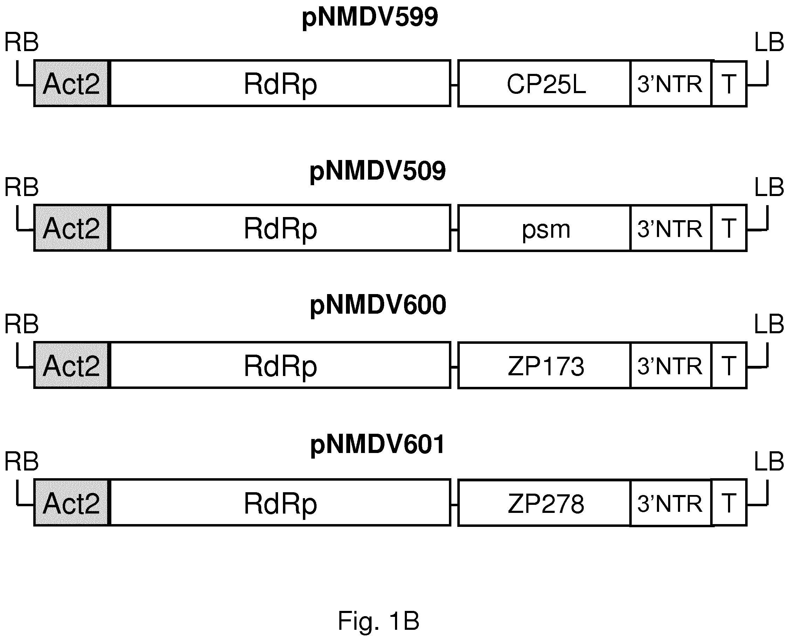

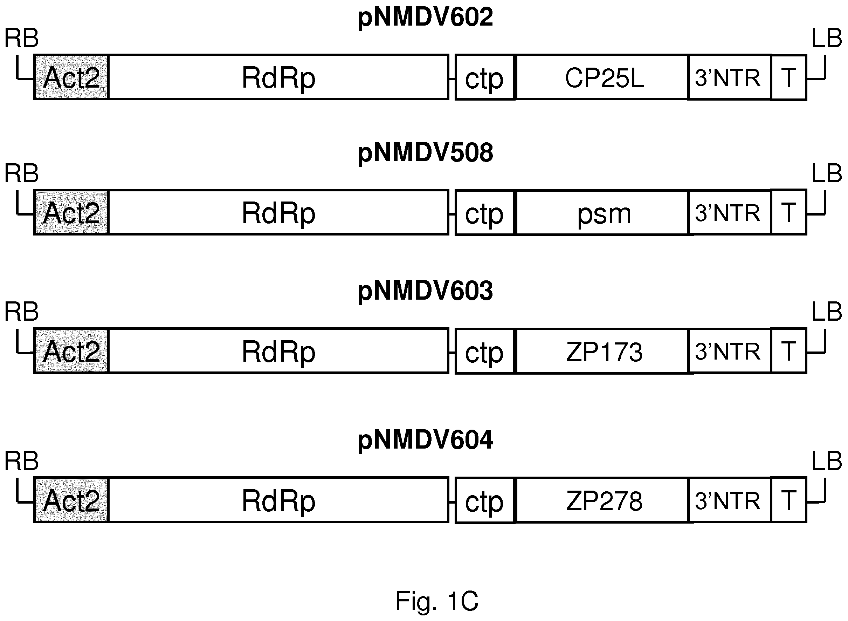

**Figure 1****. Schematic representation of T-DNA regions of used TMV-based provector modules (A) and assembled vectors (B, C).** LB - left T-DNA border, RB - right T-DNA border, Act2 and Hsp81.1 -promoters, RdRp -RNA-dependent RNA polymerase, MP -TMV movement protein, int - intron, T - nos terminator, ctp - synthetic chloroplast targeting sequence; AttP and AttB - integrase recombination sites, PhiC31 *- Streptomyces* phage C31 integrase, NLS - nuclear localization signal, PlyCP26F, PlyCP39O, CP25L, psm, ZP173 and ZP278 - coding sequences for PlyCP26F, PlyCP39O, CP25L, psm, ZP173 and ZP278 bacteriophage lysins, respectively.

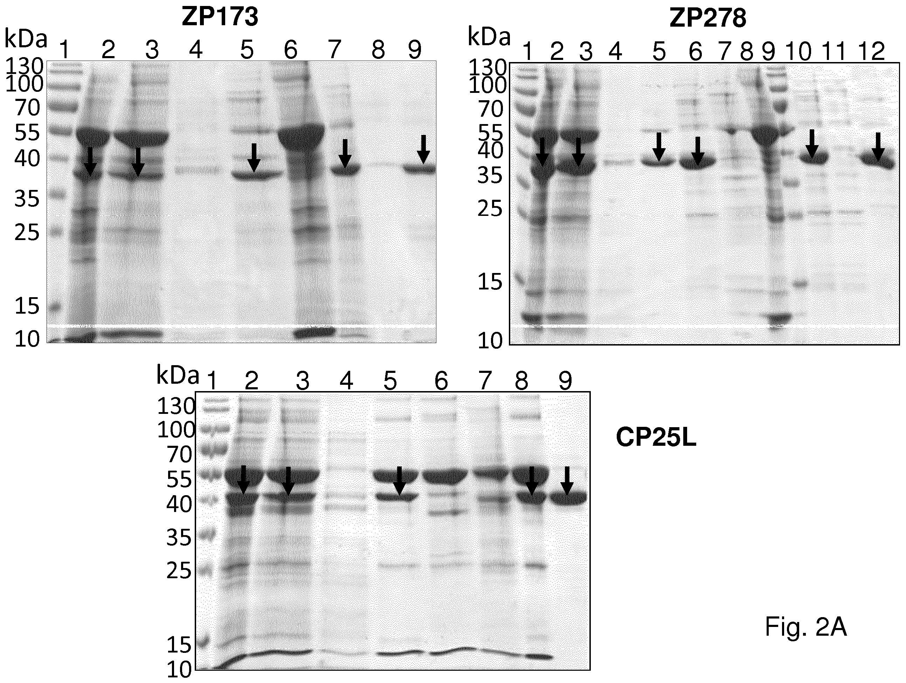

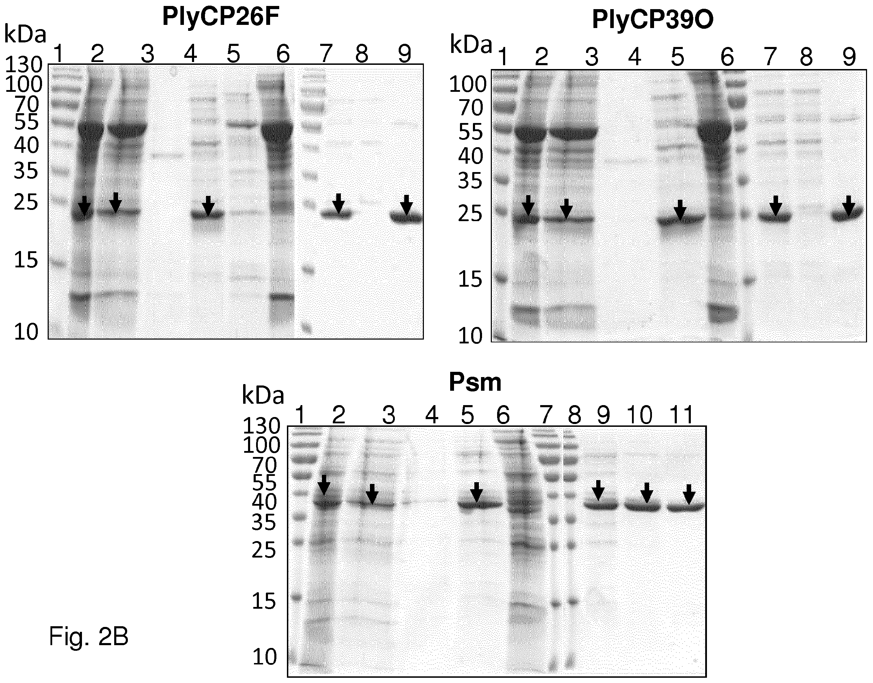

**Figure 2****. Purification of plant-produced lysins ZP173, ZP278 and CP25L (A), and PlyCP26F and PlyCP39O (B). ZP173** - lane 1- PageRuler^{™} Prestained Protein Ladder, lane 2 - extracted proteins, lane 3 - loaded proteins on Butyl sepharose, lane 4 - flow-through butyl sepharose, lane 5 - collected proteins after butyl sepharose I, lane 6 - collected proteins after butyl sepharose II, lane 7 - loaded proteins on SP sepharose, lane 8 - flow-through SP sepharose, lane 9 - collected proteins after SP sepharose. **ZP278** - lane 1- Protein Ladder, lanes 2 and 9 - extracted proteins, lane 3 - loaded proteins on butyl sepharose, lane 4 - flow through butyl sepharose, lane 5 - collected proteins after butyl sepharose I, lane 6 - collected proteins after butyl sepharose II, lane 7 - collected proteins after butyl sepharose III, lane 8 - collected proteins after butyl sepharose IV, lane 10 - loaded proteins on DEAE sepharose, lane 11 - flow through DEAE sepharose, lane 12 - collected proteins after DEAE sepharose. **CP25L** - lane 1- protein ladder, lane2 - extracted proteins, lane 3 - loaded proteins on butyl sepharose, lane 4 - flow through butyl sepharose, lane 5 - collected proteins after butyl sepharose I, lane 6 - collected proteins after butyl sepharose II, lane 7 - collected proteins after butyl sepharose III, lane 8 - loaded proteins on Q sepharose, lane 9 - collected proteins after Q sepharose. **PlyCP26F** - lane 1 and 8- protein ladder, lane 2 - extracted proteins, lane 3 - loaded proteins on butyl sepharose, lane 4 - flow through butyl sepharose, lane 5 - collected proteins after butyl sepharose I, lane 6 - collected proteins after butyl sepharose II, lane 7 - collected proteins after Butyl sepharose III, lane 9 - loaded proteins on SP sepharose, lane 10 - flow through SP sepharose, lane 11 - collected proteins after SP sepharose. **PlyCP39O** - lane 1 and 7- protein ladder, lane 2 - extracted proteins, lane 3 - loaded proteins on butyl sepharose, lane 4 - flow through butyl sepharose, lane 5 - collected proteins after butyl sepharose I, lane 6 - collected proteins after Butyl sepharose II, lane 8 - loaded proteins on SP sepharose, lane 9 - flow through SP sepharose, lane 10 - collected proteins after SP sepharose. Psm - lane 1, 7 and 8 - protein ladder, lane 2 - extracted proteins, lane 3 - loaded proteins on butyl sepharose, lane 4 - flow through butyl sepharose, lane 5 - collected proteins after butyl sepharose I, lane 6 - collected proteins after butyl sepharose II, lane 9 - loaded proteins on Q sepharose, lane 10 - collected proteins after Q sepharose I, lane 11 - collected proteins after Q sepharose II.

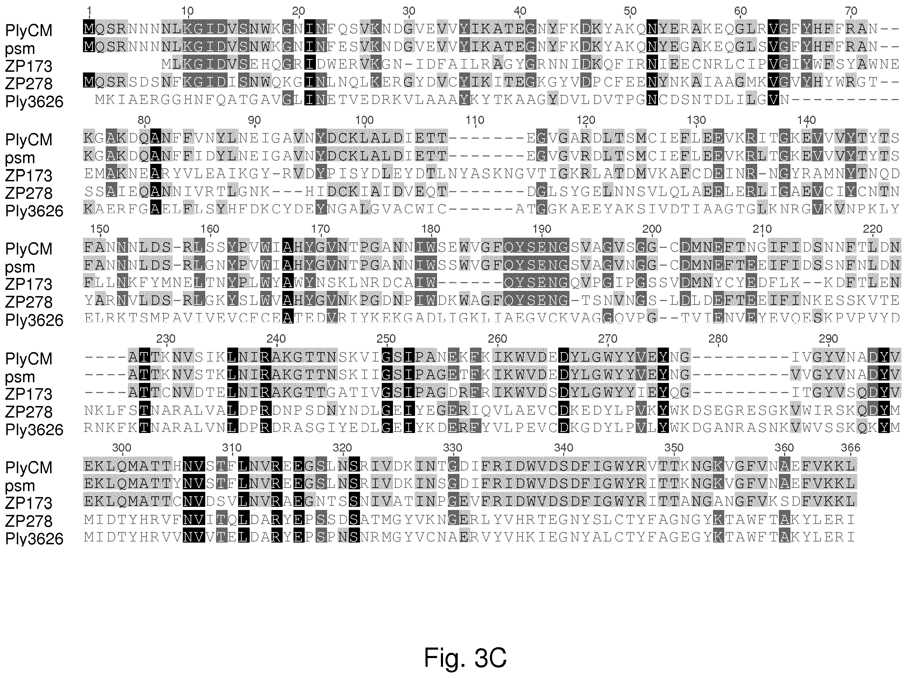

**Figure 3 (C)****. Alignment of the amino acid sequences of psm, ZP173, ZP278, Ply26F, PlyCP39O, and CP25L.** Alignment of amino acid sequences was completed using Clustal W in Geneious and shading represents conserved identical residues shared among proteins.

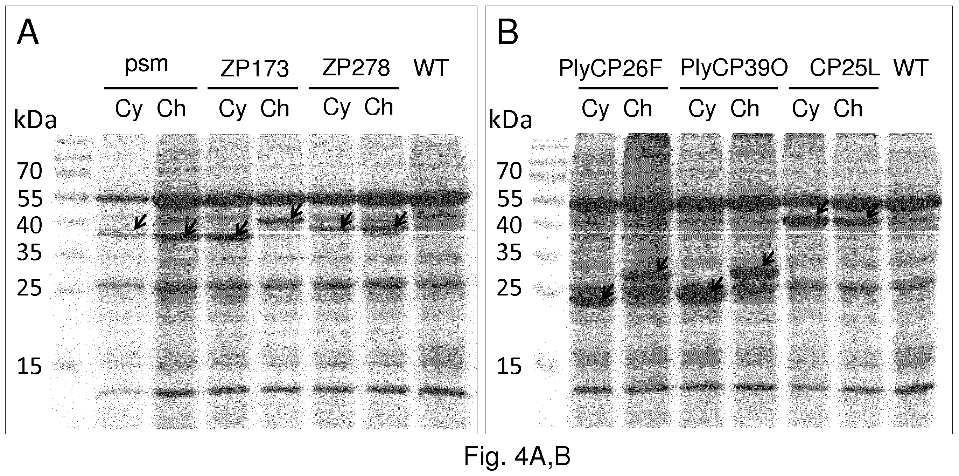

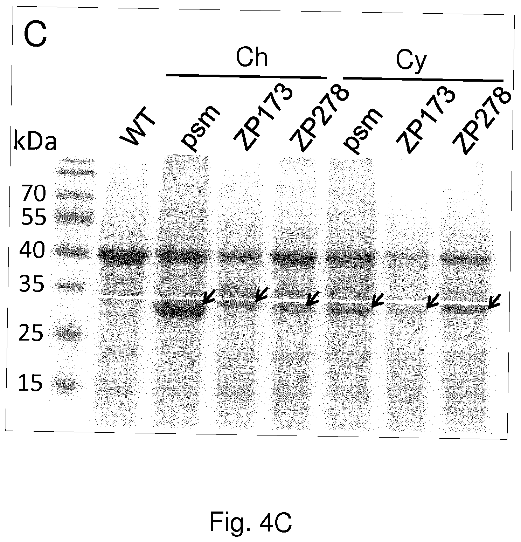

**Figure 4****. Expression of *Clostridium perfringens* bacteriophage lysins.** Coomassie stained SDS-PAGE of crude plant extracts. **A** - agro-sprayed *Nicotiana benthamiana* at 6 dps (10 dps for psm chloroplast construct). **B** - syringe - infiltrated *Nicotiana benthamiana* at 6 dpi (7 dpi for CP25L). C - agro-sprayed *Spinacia oleracea* "Frühes Riesenblatt" variety (10 dps). Crude protein extracts were prepared using Laemmli buffer. Cy - cytosol expression, Ch - chloroplast expression, WT - wild type control plants.

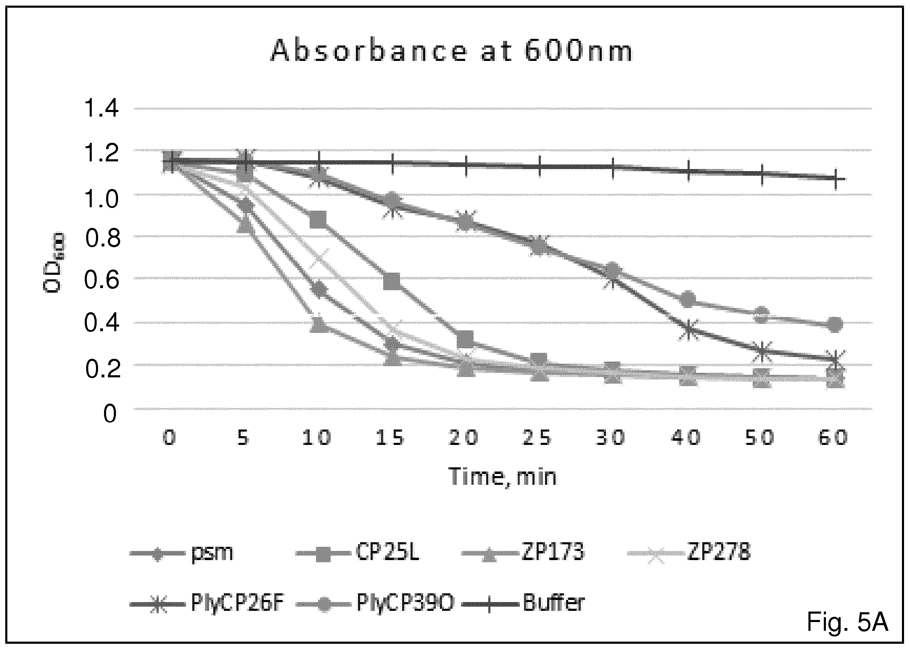

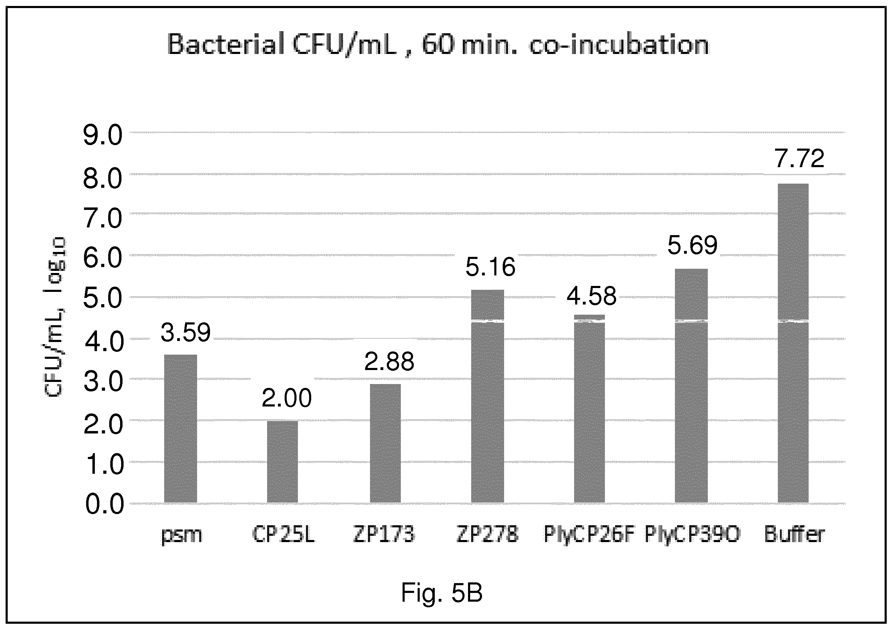

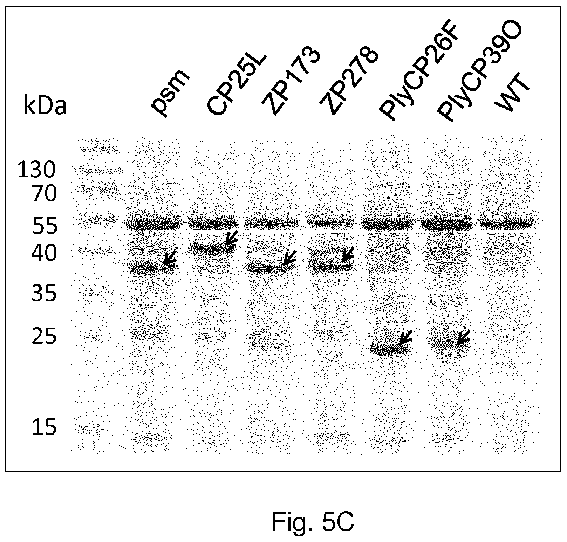

**Figure 5****. Bacteriolytic activity of crude extracts of *N. benthamiana-expressed* lysins against *C. perfringens* NCTC8237.** Crude protein extracts were prepared from agro-infiltrated *N. benthamiana* plants using extraction buffer (50 mM NaH₂PO₄, 5 mM DTT, 150 mM NaCl, (pH 7.5) and diluted to ~1 µg/µL. C. *perfringens* were grown in TSB under anaerobic conditions to OD₆₀₀=0.8 and re-suspended in 1xPBS, pH 7.3. 950 µL of bacterial suspension mixed with ~50 µL of protein extract was used for OD₆₀₀ measurements and cfu counts. Serial dilutions for cfu counts were done in 1xPBS pH 7.3 after 60 min of co-incubation with lysins. Cfu counted following o/n incubation under anaerobic conditions at 37 °C. **A** - absorbance at 600 nm. **B** - cfu counts after 1 hour of incubation with lysins. **C** - Coomassie stained SDS-PAGE of plant extracts used for experiment.

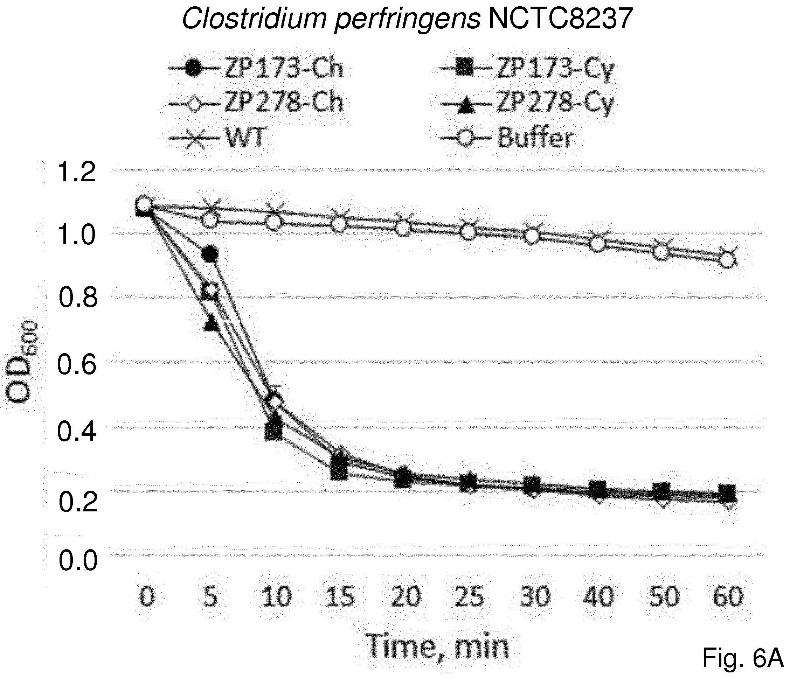

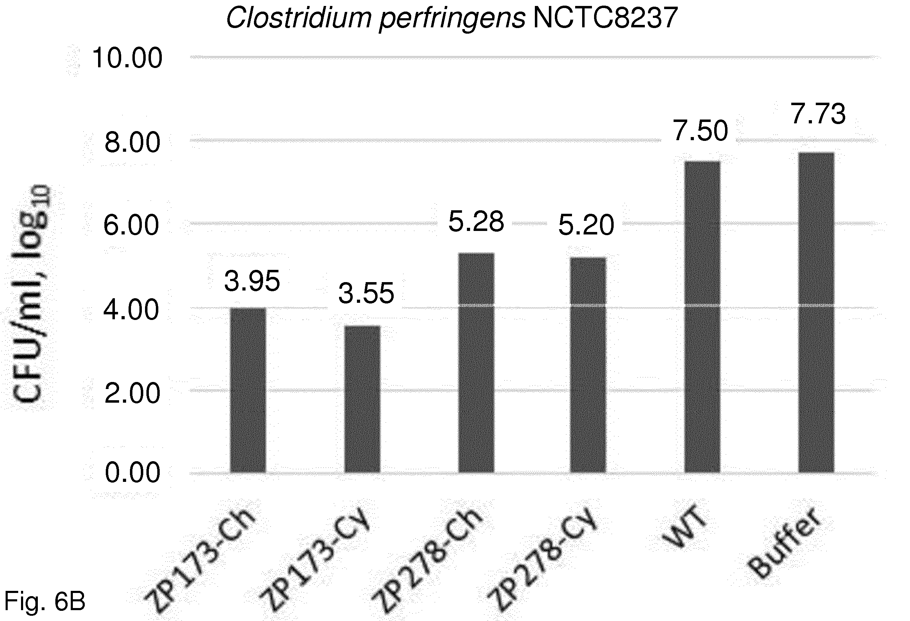

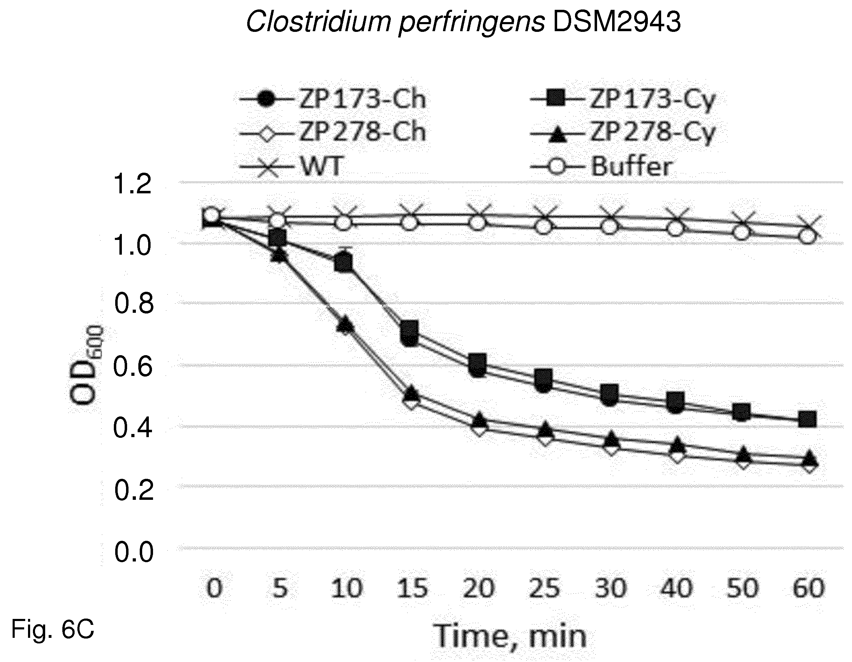

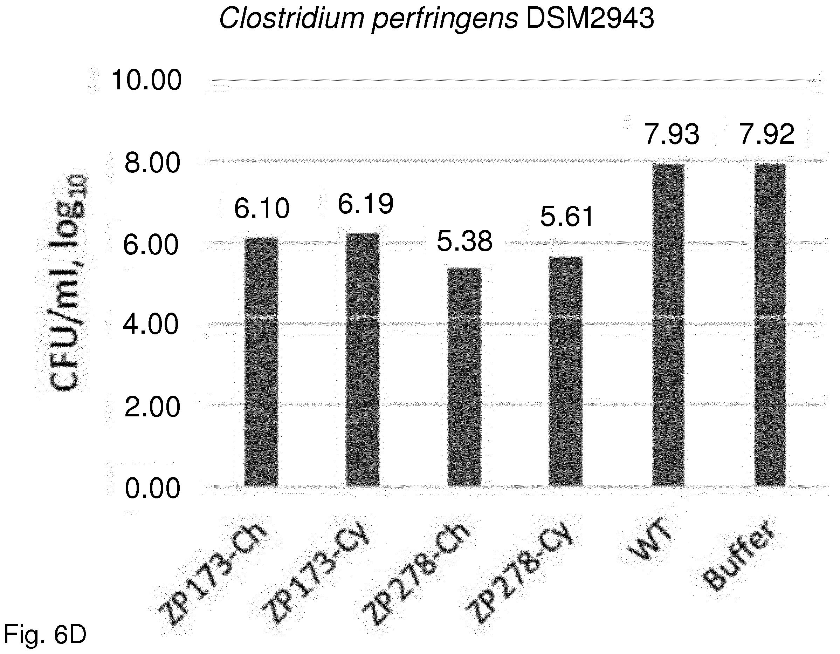

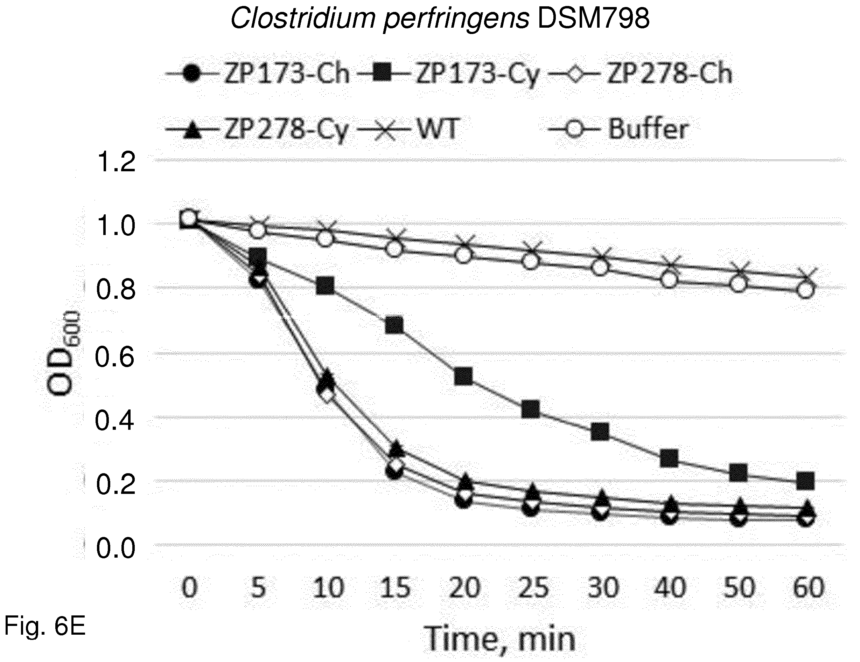

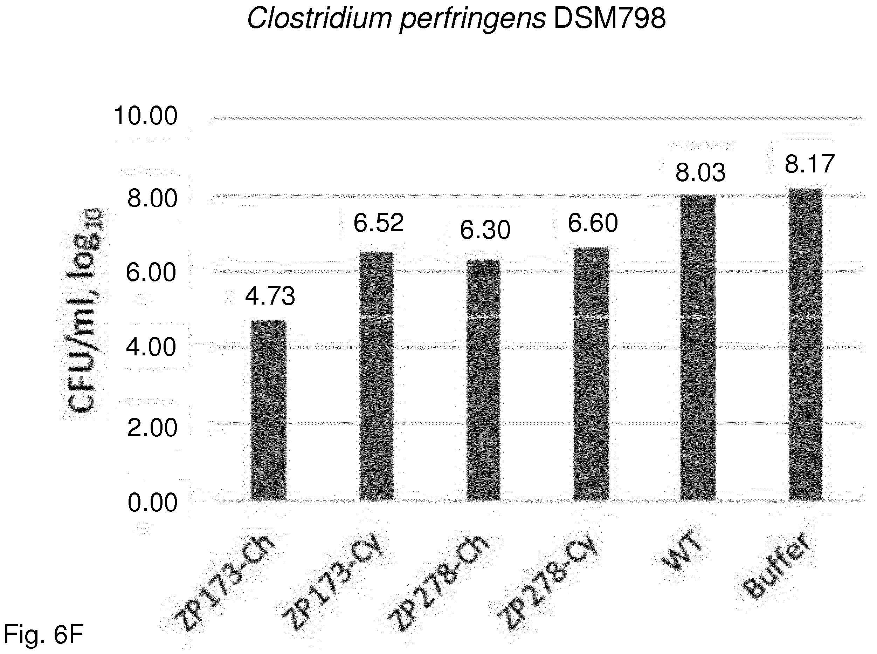

**Figure 6****. Bacteriolytic activity of spinach-expressed ZP173 and ZP278 against *Clostridium perfringens* strains NCTC8237 (A, B), DSM2943 (C, D), and DSM798 (E, F).** Crude protein extracts were prepared from agro-sprayed S. *oleracea* plants using buffer (50 mM NaH₂PO₄, 300 mM NaCl, (pH 4.5) and diluted to ~1 µg/µL. *C*. *perfringens* was grown in TSB under anaerobic conditions to OD₆₀₀=0.7, re-suspended in 1xPBS, pH 7.3. 950 µL of bacterial suspension mixed with 50 µL of protein extract was used for OD₆₀₀ measurements and cfu counts. Serial dilutions for cfu counts were done in 1xPBS pH 7.3 after 60 min of co-incubation with lysins. CFU counted on TSA plates following o/n incubation under anaerobic conditions at 37 °C. **A, C, E -** absorbance at 600 nm. **B, D, F** - cfu counts after 1 hour of incubation with lysins.

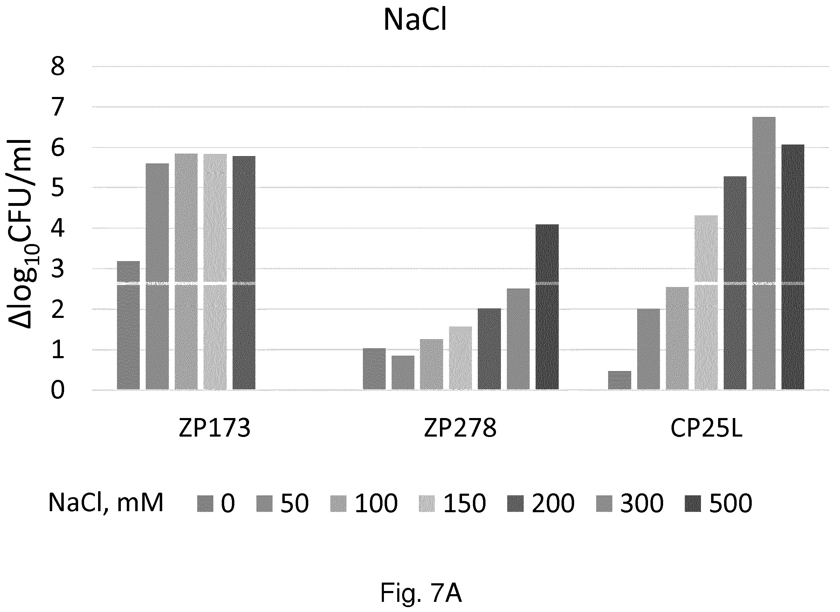

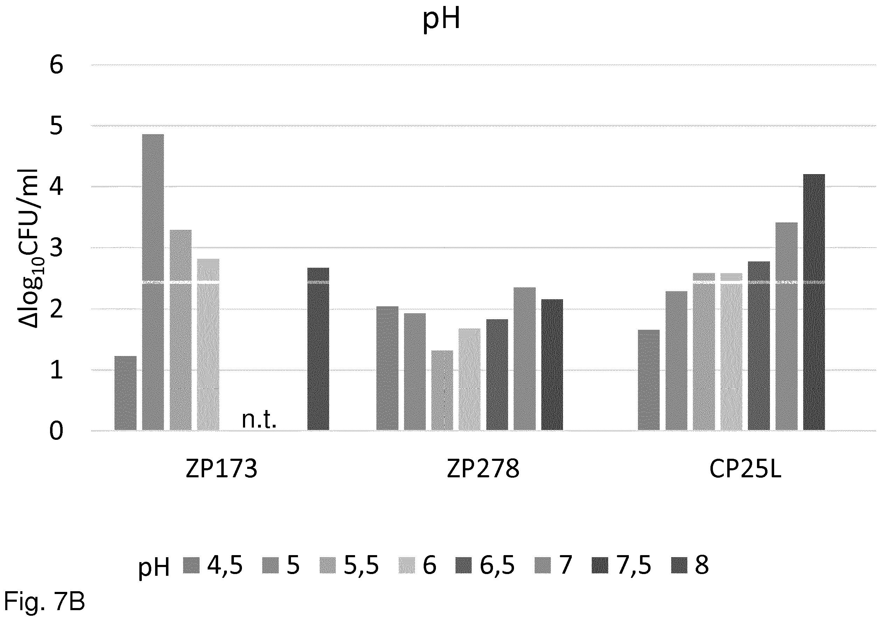

**Figure 7****. Activity of plant-expressed lysins at different concentration of NaCl and at different pH. A -** *C*. *perfringens* NCT8237 was grown in TSB under anaerobic conditions to OD₆₀₀=0.6-0.7, resuspended in citrate-Na phosphate buffer of pH 5.5 supplemented with 50 mM, 100 mM, 150 mM 150 mM, 200 mM, 300 mM and 500 mM NaCl. 1 mL of bacterial suspension mixed with 3 µg of purified ZP173, 34 µg of ZP78 or 7.5 µg of CP25L and incubated at RT for 60 min. **B** - *C*. *perfringens* NCT8237 was grown in TSB under anaerobic conditions to OD₆₀₀=0.6 - 0.76, resuspended in citrate-Na phosphate buffers pH 4.5-8.0. 1 mL of bacterial suspension mixed with 4 µg of ZP173, 27 µg of ZP278 or 5 µg of CP25L and incubated at RT for 1 h. Serial dilutions of analyzed samples were done in 1xPBS pH 7.3 and plated on TSA plates. Bacterial cfu/mL counting was done following overnight incubation under anaerobic conditions at 37 °C.

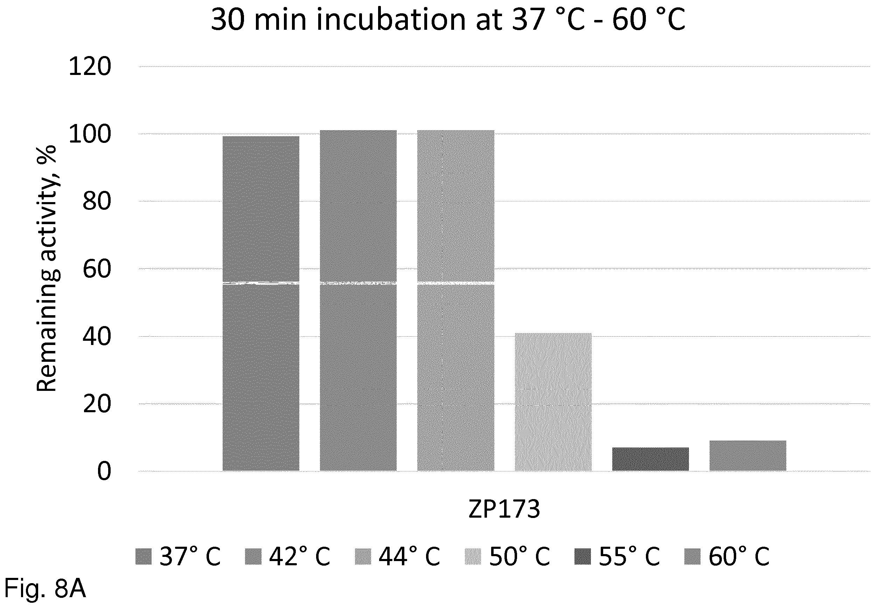

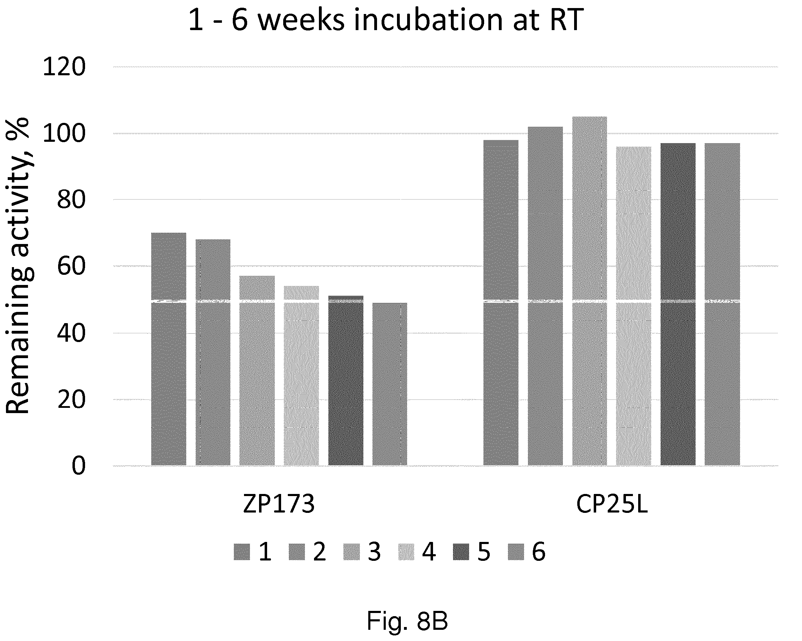

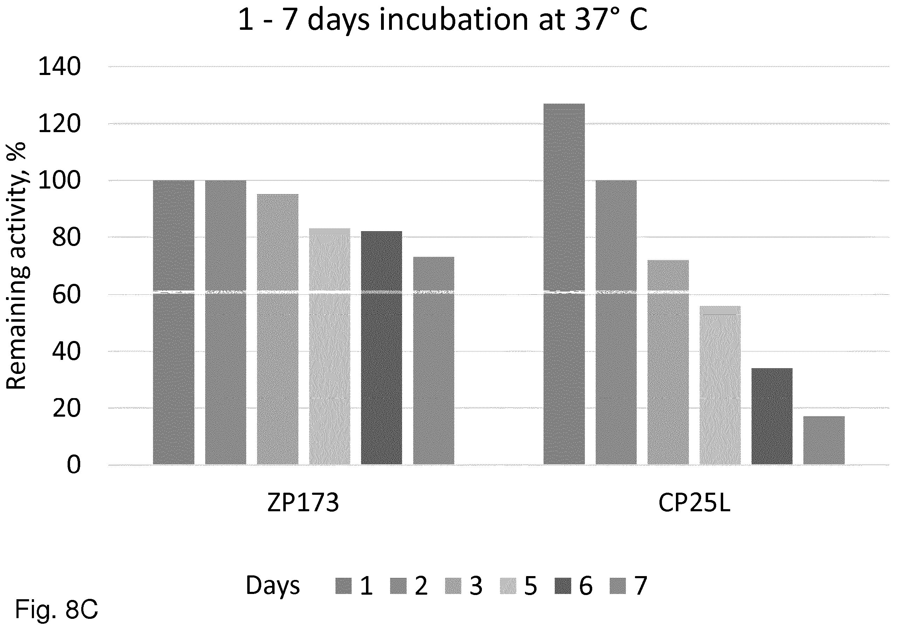

**Figure 8****. Remaining activity (%) of lysins after incubation at different** temperatures. **A** - lysins were incubated at different temperatures. **B** - lysins were stored ar RT for several weeks. **C** - lysins were incubated at 37 °C up to 7 days. **A** - lysins activity after incubation at temperatures from 37 °C to 60 °C. Purified lysins were aliquoted and incubated at temperatures indicated for 30 min. C. *perfringens* NCT8237 was grown in TSB under anaerobic conditions to OD₆₀₀=0.894, resuspended in 1xPBS of pH 7.3. 998 µL of bacterial suspension mixed with 2 µg of ZP173 and incubated at RT for 1 hour. B - lysins activity after long term storage at room temperature. Purified lysins were aliquoted and incubated at RT for 1 to 6 weeks. C. *perfringens* NCT8237 was grown in TSB under anaerobic conditions to OD₆₀₀=0.858, resuspended in 1xPBS of pH 7.3. 998 µL of bacterial suspension mixed with 2 µg of ZP173 or CP25L and incubated at RT for 1 hour. C - lysin activity after several days incubation at 37 °C. Purified lysins were aliquoted and incubated at 37 °C for 1 to 7 days. C. *perfringens* NCT8237 was grown in TSB under anaerobic conditions to OD₆₀₀=0,858, resuspended in 1xPBS of pH 7.3. 998 µL of bacterial suspension mixed with 2 µg of ZP173 or CP25L and incubated at RT for 1 hour. Serial dilutions of analyzed samples were done in 1xPBS pH 7.3 and plated on TSA plates Bacterial cfu was counted following o/n incubation under anaerobic conditions at 37 °C.

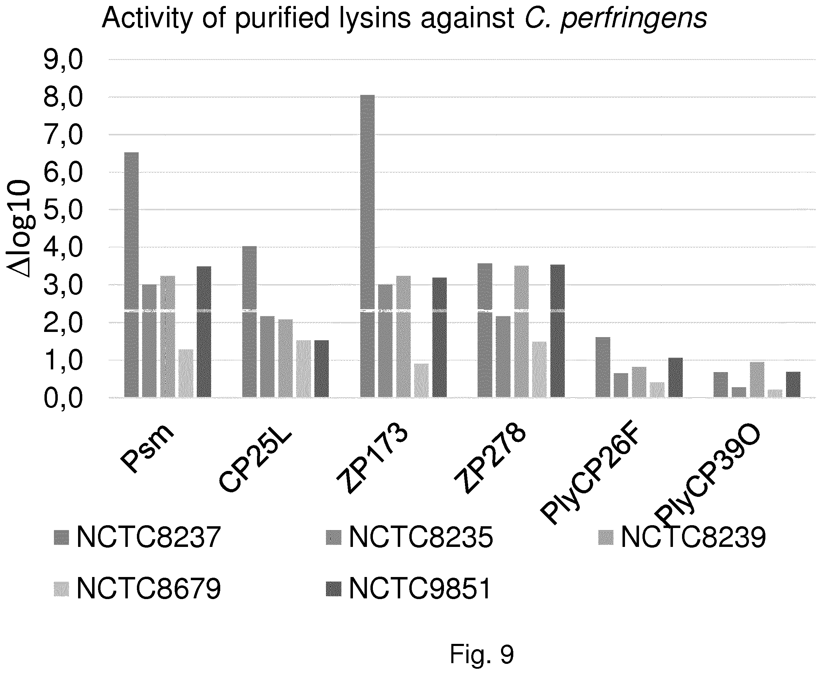

**Figure 9****. Bacteriolytic activity of purified lysins against different *C. perfringens* food strains.** Purified proteins were dissolved to concentration appr. 0,3-0,9 µg/µL. *C*. *perfringens* were grown to OD₆₀₀=0.8 in TSB anaerobically, centrifuged and resuspended in 20 mM citrate-phosphate buffer supplemented with 50 mM NaCl, pH 5,5. 967 µL of bacterial suspension was mixed with 10 µg protein and incubated at RT. Samples for CFU counting were taken after 1 h co-incubation. Serial dilutions of analyzed samples were done in a resuspension buffer and 30 µL of each sample plated on TSA plates: undiluted, diluted 10, 10², 10³, 10⁴, and 10⁵ times. Images of plates taken following o/n incubation under anaerobic conditions at 37 °C.

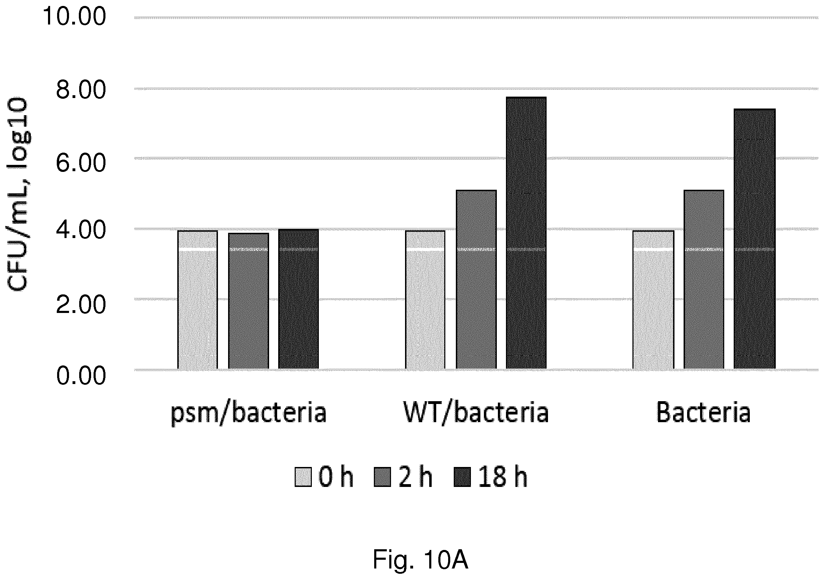

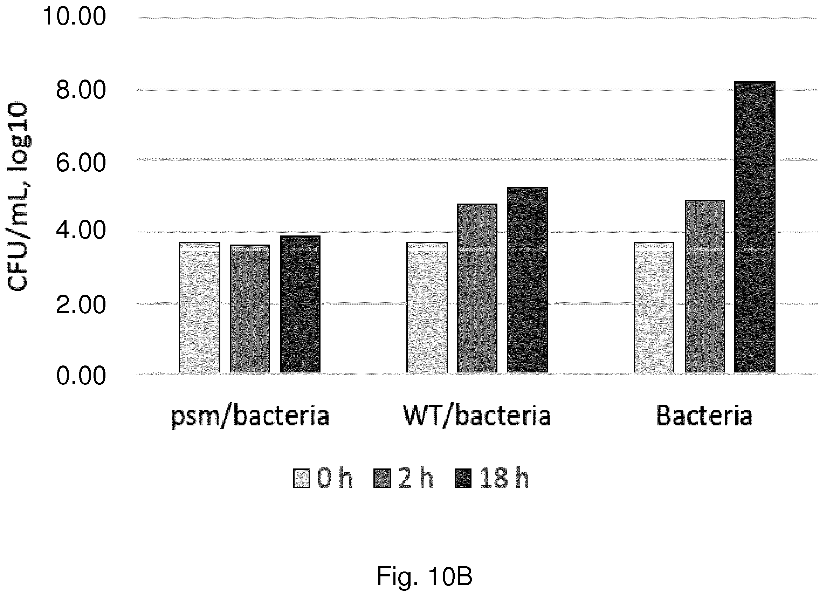

**Figure 10****. Activity of crude** psm **extract against *C. perfringens* NCTC8237 in cooked turkey (A) and beef (B) mince.** Crude psm extract was prepared from agro-infiltrated N. *benthamiana* plants harvested 6 dpi following the standard procedure. Protein extract was diluted to conc. ~0.44 µg/µL. C. *perfringens* NCTC8237 strain was grown in TSB (tryptic soy broth) under anaerobic conditions to OD₆₀₀=0.265. 100 µL of bacteria and 700 µL≈300 µg of protein extract was added to 10 g of cooked meat and vortexed well. To equalize the volume of all samples, 700 µL of 1xPBS, pH 7.3 was added to meat/bacteria sample. All the samples were placed into a 6-well plate and incubated under anaerobic conditions at room temperature. Serial dilutions of analyzed samples were done in 1xPBS pH 7.3 and plated on TSA plates. Bacterial cfu was counted following o/n incubation under anaerobic conditions at 37 °C.

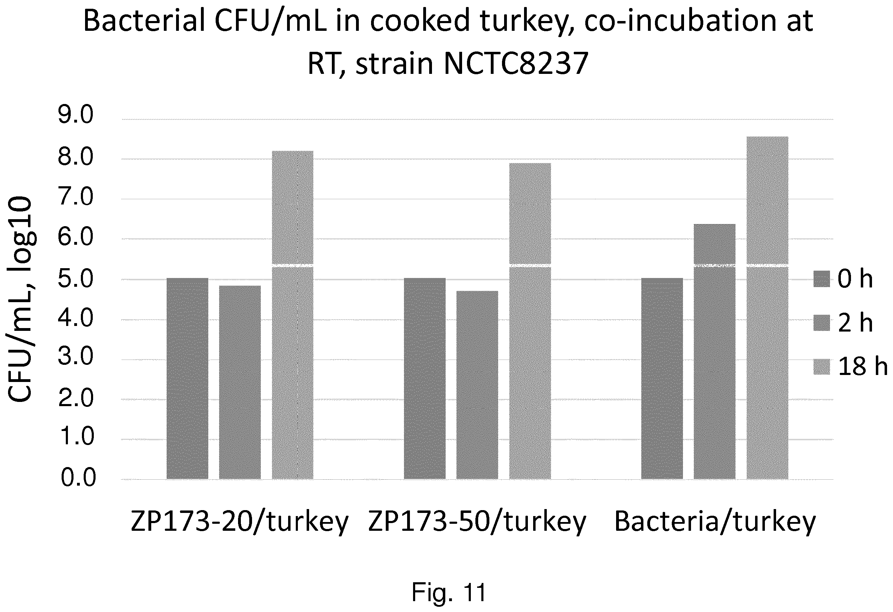

**Figure 11****. Activity of purified ZP173 against *C. perfringens* NCTC8237 in cooked turkey mince.** *C*. *perfringens* NCT8237 was grown in TSB under anaerobic conditions to OD₆₀₀= 0.24. 10 g of cooked turkey breast meat was combined with 100 µL of bacteria, 20 µg or 50 µg of ZP173 and 3 mL sterile water, mixed well and incubated in a 6-well plate at RT anaerobically. Samples for cfu counting were taken just before starting the experiment and after 2 h and 18 h co-incubation. Serial dilutions of analyzed samples were done in 1xPBS pH 7.3 and plated on TSA plates Bacterial cfu was counted following o/n incubation under anaerobic conditions at 37 °C.

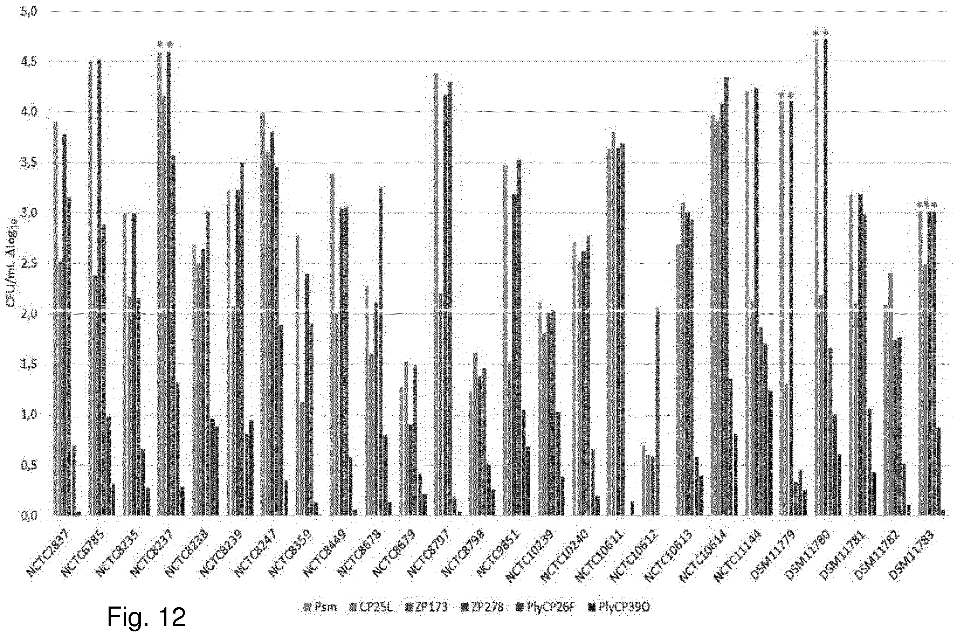

**Figure 12****. Killing of *C. perfringens* food strains by *N. benthamiana-expressed* lysins.** CFU/mL Δlog10 of different *C*. *perfringens* strains treated with each lysin separately. C. *perfringens* strains were grown in TSB under anaerobic conditions to OD600~ 0.8 and re-suspended in citrate-phosphate buffer, 50 mM NaCl, pH 5.5. Lysins were added to bacterial suspension at final concentration of 10 µg/ml. Serial dilutions for cfu counts were done in PBS, pH 7.3 after 60 min. of co-incubation with lysins. * - no colonies were detected on plates.

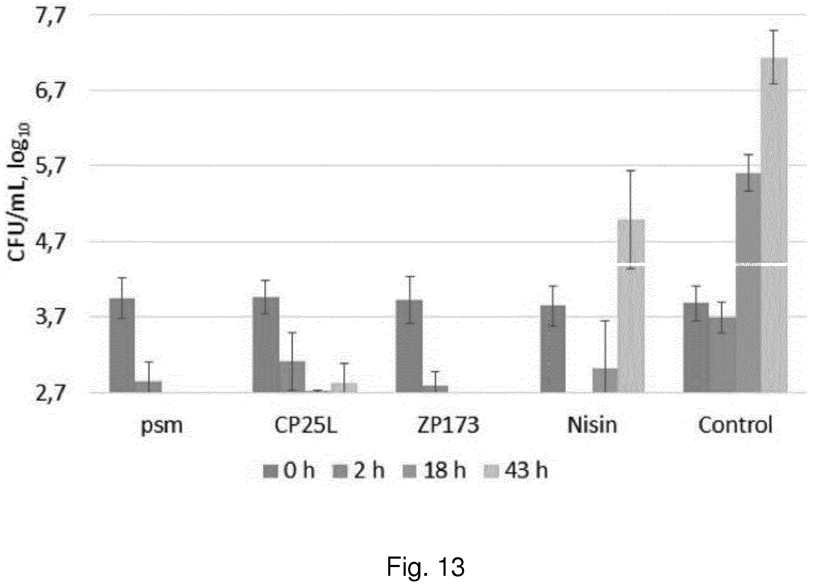

**Figure 13****. Activity of purified lysins in *C*. perfringens-contaminated turkey at room temperature.** 4 log10 cfu of *C*. *perfringens* NCTC8237 were mixed with 10 g of chopped cooked turkey meat, 3 ml of citrate-phosphate buffer supplemented with (final concentration of 1.5%), pH 5.5 and 2.5 µg/ml of purified lysin or 5 µg/ml of nisin. 0 h - cfu counts of samples before addition of lysins or nisin. 2 h, 18 h and 43 h - cfu counts of samples, incubated at RT anaerobically for the indicated time. Data are the mean ± SD of four independent experiments.

### DETAILED DESCRIPTION OF THE INVENTION

Bacteriophage endolysins (endolysins are also referred to herein as "lysins") can kill bacterial cells by various mechanisms, such as by hydrolyzing bacterial cell wall components. Lysins active against Gram-positive bacteria are generally modular monomeric proteins of 250-400 amino acid residues in length, with an N-terminal enzymatic (or catalytic) domain and a C-terminal binding domain. There are at least four groups of lysins based on the cleavage site of their catalytic domain that acts on bacterial cell wall structures:
1) N-acetylmuramidases (lysozymes) cleave the β-1-4-glycosidic bond between NAG-NAM;
2) N-acetyl-β-D-glucosaminidases (glycosidases) cleave β-1-4-glycosidic bond between NAM-NAG;
3) N-acetylmuramoyl-L-alanine amidases cleave the amide bond between sugar and peptide (EC 3.5.1.28)
4) L-alanoyl-D-glutamate endopeptidases and interpeptide bridge-specific endopeptidases cleave the peptide moiety of the cell wall peptidoglycan. Lysins used in the present invention can be any one or more of these types 1) to 4).

The invention makes use of one or more lysin that is/are active against *Clostridium,* notably pathogenic *Clostridium.* Examples of pathogenic *Clostridium* are:
*Clostridium botulinum:* produces botulinum toxin; causes botulism;
*Clostridium difficile:* disbacteriosis after antibiotic therapy; pseudomembranous colitis
*Clostridium perfringens:* a wide range of symptoms, from food poisoning to gas gangrene;
*Clostridium tetani:* produces tetanus toxin; causes tetanus;
*Clostridium sordelii:* can cause a fatal infection in exceptionally rare cases after medical abortions.

The invention makes use of one or more lysin active against any one of the above species of *Clostridium.* Preferably, the invention makes use of one or more lysin that is/are active against *Clostridium perfringens* or *Clostridium difficile.* Preferably, the one or more lysin is active against *Clostridium perfringens.* Serotypes of *Clostridium perfringens* and toxins thereof are as follows: there are five serotypes A, B, C, D and E depending on the type of toxins (enterotoxins): α, β, ε, I (alpha-, beta-, epsilon-, and iota-toxins). C. *perfringens* enterotoxin (CPE) is the main virulent factor that initiates many critical gastrointestinal (GI) diseases. CPE causes the breakdown of cell membrane permeability often resulting in the cell death. *Clostridium perfringens* type A is a normal inhabitant of GI tract in pigs. Most preferably, the one or more lysin is active against *Clostridium perfringens* Type A.

The expression "active against *Clostridium"* means that the lysin has bacteriolytic activity against the *Clostridium.* Bacteriolytic activity against *Clostridium* may be tested experimentally e.g. using the turbidity assay used in the experimental section or by comparing the colony-forming units (cfu) of a *Clostridium* sample in the presence and absence of a lysin to be tested as described in the experimental section.

In the composition and method of the invention, at least one lysin is employed. More than one lysin, such as two, three, four or more may be used, e.g. as a mixture, in the method, process or composition of the invention. If two or more lysins are combined, it is preferred to combine lysins having different target specificities as determined by the binding domains of the lysins for achieving broad *anti- Clostridium* activity and/or for avoiding development of resistance to the lysin by mutation of the *Clostridium.* Alternatively or additionally, two or more lysins are used in the method or composition that have different catalytic activities among the four types 1) to 4) listed above. For example, a lysin of type 1) may be combined with a lysin of type 3).

In one embodiment of the invention, at least one of said at least one lysin is or comprises a muramidase-containing lysin of above type 1), namely ZP173 having an amino acid sequence comprising or consisting of the amino acid sequence of SEQ ID NO: 2 or a derivative of this lysin. Other endolysisin that, however, are not part of this invention are Psm having an amino acid sequence comprising or consisting of the amino acid sequence of SEQ ID NO: 1 and ZP278 having an amino acid sequence comprising or consisting of the amino acid sequence of SEQ ID NO: 3. Accession numbers and origins of these lysins are given in the Examples. Derivatives of these lysins may have at least 33% bacteriolytic activity of the lysin it is a derivative of, which may be tested experimentally, e.g. the turbidity assay used in the examples.

Another endolysin that is not part of the invention is or comprises a lysin having N-acetylmuramoyl-L-alanine amidase activity. Such lysin may be selected from CP25L having an amino acid sequence comprising or consisting of the amino acid sequence of SEQ ID NO: 4, PlyCP26F having an amino acid sequence comprising or consisting of the amino acid sequence of SEQ ID NO: 5, and PlyCP39O having an amino acid sequence comprising or consisting of the amino acid sequence of SEQ ID NO: 6, or a derivative of any of these lysins. Derivatives of these lysins may have at least 33% bacteriolytic activity of the endolysin it is a derivative of. The derivative is a derivative of that lysin (parent lysin) among CP25L, PlyCP26F and PlyCP39O to which it has the highest sequence identity.

Psm, ZP173 and ZP278, CP25L, PlyCP26F and PlyCP39O having amino acid sequences consisting of the amino acid sequences of the SEQ ID NOs given above are also referred to herein as parent lysins.

A derivative (of a parent) lysin has a sequence identity of at least 90%, preferably at least 95% and most preferably at least 97% to an amino acid sequence of any one of SEQ ID NO: 1, SEQ ID NO: 2, SEQ ID NO: 3, SEQ ID NO: 4, SEQ ID NO: 5, or SEQ ID NO: 6; or said derivative has from 1 to 30, preferably from 1 to 20, more preferably from 1 to 15, and even more preferably from 1 to 10 amino acid substitutions, insertions and/or deletions compared to an amino acid sequence of any one of SEQ ID NO: 1, SEQ ID NO: 2, SEQ ID NO: 3, SEQ ID NO: 4, SEQ ID NO: 5, or SEQ ID NO: 6.

Accordingly, the amino acid sequence of a derivative lysin has a sequence identity of at least 90%, preferably at least 95% and most preferably at least 97% to the amino acid sequence of any one of SEQ ID NO: 1, SEQ ID NO: 2, SEQ ID NO: 3, SEQ ID NO: 4, SEQ ID NO: 5, or SEQ ID NO: 6; or the amino acid sequence of the derivative may have from 1 to 30, preferably from 1 to 20, more preferably from 1 to 15, and even more preferably from 1 to 10 amino acid substitutions, insertions and/or deletions compared to the amino acid sequence of any one of SEQ ID NO: 1, SEQ ID NO: 2, SEQ ID NO: 3, SEQ ID NO: 4, SEQ ID NO: 5, or SEQ ID NO: 6. Derivatives of the parent lysins are also lysins in the sense of the invention.

Among derivatives of Psm, ZP173 and ZP278, some amino acid residues are relevant or useful for the functionality of the lysin and should not be changed compared to the parent lysins. Instead, in preferred embodiments, the amino acid residue(s) corresponding to
residue 10 of SEQ ID NO: 1 is Lys,
residue 11 of SEQ ID NO: 1 is Gly;
residue 12 of SEQ ID NO: 1 is Ile;
residue 13 of SEQ ID NO: 1 is Asp;
residue 21 of SEQ ID NO: 1 is Ile or Leu;
residue 27 of SEQ ID NO: 1 is Lys;
residue 41 of SEQ ID NO: 1 is Gly;
residue 46 of SEQ ID NO: 1 is Asp;
residue 52 of SEQ ID NO: 1 is Asn;
residue 63 of SEQ ID NO: 1 is Val;
residue 64 of SEQ ID NO: 1 is Gly;
residue 66 of SEQ ID NO: 1 is Tyr;
residue 79 of SEQ ID NO: 1 is Ala;
residue 86 of SEQ ID NO: 1 is Leu or Ile;
residue 100 of SEQ ID NO: 1 is Leu, Val or Ile;
residue 101 of SEQ ID NO: 1 is Asp or Glu;
residue 123 of SEQ ID NO: 1 is Asp or Glu;
residue 126 of SEQ ID NO: 1 is Arg;
residue 129 of SEQ ID NO: 1 is Gly;
residue 135 of SEQ ID NO: 1 is Tyr;
residue 143 of SEQ ID NO: 1 is Asn;
residue 149 of SEQ ID NO: 1 is Leu or Ile;
residue 152 of SEQ ID NO: 1 is Tyr;
residue 155 of SEQ ID NO: 1 is Trp;
residue 159 of SEQ ID NO: 1 is Tyr;
residue 169 of SEQ ID NO: 1 is Ile;
residue 170 of SEQ ID NO: 1 is Trp;
residue 177 of SEQ ID NO: 1 is Gln;
residue 178 of SEQ ID NO: 1 is Tyr;
residue 179 of SEQ ID NO: 1 is Ser;
residue 180 of SEQ ID NO: 1 is Glu;
residue 181 of SEQ ID NO: 1 is Asn;
residue 182 of SEQ ID NO: 1 is Gly;
residue 189 of SEQ ID NO: 1 is Gly;
residue 192 of SEQ ID NO: 1 is Asp, Glu, or Asn, preferably Asp;
residue 198 of SEQ ID NO: 1 is Asp, Glu, or Asn, preferably Glu;
residue 213 of SEQ ID NO: 1 is Thr;
residue 221 of SEQ ID NO: 1 is Leu;
residue 224 of SEQ ID NO: 1 is Arg;
residue 235 of SEQ ID NO: 1 is Gly;
residue 237 of SEQ ID NO: 1 is Ile;
residue 240 of SEQ ID NO: 1 is Gly;
residue 251 of SEQ ID NO: 1 is Asp;
residue 260 of SEQ ID NO: 1 is Tyr;
residue 270 of SEQ ID NO: 1 is Asp;
residue 282 of SEQ ID NO: 1 is Asn;
residue 283 of SEQ ID NO: 1 is Val;
residue 287 of SEQ ID NO: 1 is Leu;
residue 290 of SEQ ID NO: 1 is Arg;
residue 292 of SEQ ID NO: 1 is Glu;
residue 297 of SEQ ID NO: 1 is Ser; and/or
residue 306 of SEQ ID NO: 1 is Gly.

Among derivatives of CP25L, PlyCP26F and PlyCP39O, some amino acid residues may be relevant for the functionality of the lysin and should therefore not be changed compared to the parent lysins. Instead, the amino acid residue(s) corresponding to
residue 12 of SEQ ID NO: 4 is Gly;
residue 45 of SEQ ID NO: 4 is Gly;
residue 71 of SEQ ID NO: 4 is Gly;
residue 72 of SEQ ID NO: 4 is Ala;
residue 121 of SEQ ID NO: 4 is Gly;
residue 149 of SEQ ID NO: 4 is Gly;
residue 188 of SEQ ID NO: 4 is Asn;
residue 189 of SEQ ID NO: 4 is Arg;
residue 209 of SEQ ID NO: 4 is Ile, Leu or Val;
residue 226 of SEQ ID NO: 4 is Ile, Leu or Val;
residue 267 of SEQ ID NO: 4 is Ala;
residue 273 of SEQ ID NO: 4 is Pro;
residue 282 of SEQ ID NO: 4 is Arg;
residue 283 of SEQ ID NO: 4 is Val;
residue 287 of SEQ ID NO: 4 is Arg;
residue 311 of SEQ ID NO: 4 is Glu or Asp, preferably Glu;
residue 312 of SEQ ID NO: 4 is Lys;
residue 317 of SEQ ID NO: 4 is Ile, Leu or Val;
residue 323 of SEQ ID NO: 4 is Tyr; and/or
residue 330 of SEQ ID NO: 4 is Ile, Leu or Val, preferably Ile. These lysins are not according to the invention.

An amino acid residue x1 in an amino acid sequence x corresponding to a residue y1 of SEQ ID NO: y means that the amino acid sequence x and SEQ ID NO: y are aligned for optimum sequence identity over the entire length of SEQ ID NO: y and the residue x1 corresponding to amino acid residue y1 of SEQ ID NO: y is that residue that is aligned with residue y1.

Herein, the determination of amino acid sequence identities is done using Align Sequences Protein BLAST (BLASTP 2.6.1+) (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.).

A derivative of a parent lysin may comprise an additional N- or C-terminal amino acid sequence stretch such as purification tags, e.g. as a His-tag of 6 or more contiguous histidine residues. In one embodiment, the derivative has, preferably, no N-terminal amino acid residue addition.

A lysin or derivative thereof according to the invention may be produced by known methods of protein expression in a standard expression system. For producing the lysin or derivative, a nucleotide sequence encoding it may be expressed in a suitable host organism. Methods usable for producing and purifying a protein of interest have been described in the prior art and any such methods may be used. An *E. coli* expression system as generally known in the art may, for example, be used. If a eukaryotic expression system is used, one or more introns may be inserted in the coding sequence of the lysin.

Particularly efficient expression methods are plant expression systems that are also known in the prior art. Plant expression systems for expressing a lysin according to the invention are described in the Examples. A possible way of achieving expression of a nucleotide sequence of interest in plants is the use of self-replicating (viral) replicons containing the nucleotide sequence encoding the lysin. Plant viral expression systems have been described in many publications, such as in WO2012019660, WO2008028661, WO2006003018, WO2005071090, WO2005049839, WO2006012906, WO02101006, WO2007137788 or WO02068664 and many more publications are cited in these documents. Various methods for introducing a nucleic acid molecule, such as a DNA molecule, into a plant or plant part for transient expression are known. Agrobacteria may be used for transfecting plants with the nucleic acid molecule (vector) or nucleic acid construct e.g. by agroinfiltration or spraying with agrobacterial suspensions. For references, see WO 2012019660, WO 2014187571, or WO 2013149726.

In embodiments wherein strong expression of the lysin as a protein of interest is desired, a nucleic acid construct containing a nucleotide sequence encoding the lysin may encode a viral vector that can replicate in plant cells to form replicons of the viral vector. In order to be replicating, the viral vector and the replicons may contain an origin of replication that can be recognized by a nucleic acid polymerase present in plant cells, such as by the viral polymerase expressed from the replicon. In case of RNA viral vectors (referred to as "RNA replicons"), the replicons may be formed by transcription under the control of a promoter active in plant cells, from the DNA construct after the latter has been introduced into plant cell nuclei. In case of DNA replicons, the replicons may be formed by recombination between two recombination sites flanking the sequence encoding the viral replicon in the DNA construct, e.g. as described in WO00/17365 and WO 99/22003. If the replicon is encoded by the DNA construct, RNA replicons are preferred. Use of DNA and RNA viral vectors (DNA or RNA replicons) has been extensively described in the literature over the years. Some examples are the following patent publications: WO2008028661, WO2007137788, WO 2006003018, WO2005071090, WO2005049839, WO02097080, WO02088369, WO02068664. Examples of DNA viral vectors are those based on geminiviruses. For the present invention, viral vectors or replicons based on plant RNA viruses, notably those based on plus-sense single-stranded RNA viruses may be preferably used. Accordingly, the viral replicon may be a plus-sense single-stranded RNA replicon. Examples of such viral vectors are those based on tobacco mosaic virus (TMV) and potexvirus X (PVX). "Based on" means that the viral vector uses the replication system such as the replicase and/or other proteins involved in replication of these viruses. Potexvirus-based viral vectors and expression systems are described in EP2061890 or WO2008/028661.

The lysin may be expressed in a multi-cellular plant or a part thereof, notably a higher plant or parts thereof. Both monocot and dicot (crop) plants can be used. Common plants usable for expressing the protein of interest include *Nicotiana benthamiana, Nicotiana tabacum,* spinach, *Brassica campestris, B. juncea,* beets *(Beta vulgaris),* cress, arugula, mustard, Strawberry, *Chenopodium capitatum,* lettuce, sunflower, cucumber, Chinese cabbage, cabbage, carrot, green onion, onion, radish, lettuce, field peas, cauliflower, broccoli, burdock, turnip, tomato, eggplant, squash, watermelon, prince melon, and melon. Preferred plants are spinach, chard, beetroot, carrot, sugar beet, *Nicotiana tabacum,* and *Nicotiana benthamiana.* Expression in edible plants may be used for preventing contamination of the plants or food made therefrom with *Clostridium.* In one embodiment, plants are used that do not normally enter the human or animal food chain such as *Nicotiana* species such as N. *tabacum* and *N. benthamiana.*

Generally, the lysin as a protein of interest is expressed in the cytosol of cells of the plants or plant parts. In this case, no signal peptide directing the protein of interest into a particular compartment is added to the enzyme. Alternatively, the protein of interest can be expressed in or targeted into chloroplasts of the plants; in the latter case, an N-terminal pre-sequence, generally referred to as plastid transit peptide or chloroplast targeting peptide, is added to the N-terminal or C-terminal end, preferably the C-terminal end, of the lysin as the protein of interest.

In the process of producing a composition comprising at least one endolysin, a lysin is, in the first step, expressed in a plant or cells of a plant, such as an edible plant. In the next step, plant material containing expressed lysin from a plant having expressed the endolysin is harvested. Plant material may e.g. be leaves, roots, tubers, or seeds, or a crushed, milled or comminuted product of leaves, roots, tubers, or seeds. In step (iii), the lysin is extracted from the plant material using an aqueous buffer. This may include that the plant material is homogenized and insoluble material may be removed by centrifugation or filtration. Soluble components including the lysin will be extracted into the aqueous buffer to produce a lysin solution in the aqueous buffer. The aqueous buffer may contain an inorganic or organic acid or salts thereof and may have a pH as defined below for the aqueous solution as a composition of the invention. Further, the aqueous buffer may contain salt and/or a sulfhydryl compound as also defined below for the aqueous solution as a composition of the invention. If a relatively pure lysin composition is desired, the lysin solution in the aqueous buffer may be further purified by removing undesired components in step (iv) according to known methods of protein purification.

The inventors have found an efficient way of further purifying a lysin expressed in plants. Such method of purification may comprise the following steps:
(a) extraction of expressed lysin from plant material, e.g. using an aqueous buffer as defined above,
(b) removal of insoluble material from the extract to obtain a lysin solution in the aqueous buffer, optionally followed by concentrating the solution, and
(c) subjecting the lysin solution in the aqueous buffer to hydrophobic interaction chromatography (HIC), optionally followed by desalting or buffer exchange.

As a further purification step, an ion-exchange chromatography step may be used after step (c). The ion-exchange chromatography may be a cation or anion exchange chromatography step. For lysin ZP173, PlyCP26F, PlyCP39O and derivatives thereof, cation exchange chromatography is preferred. For lysins ZP278, CP25L and psm and derivatives thereof, anion exchange chromatography is preferred.

If lysins are expressed in edible plants, crude protein extracts from the edible plants or semi-purified concentrates may be used for preventing or reducing contamination of an object such as food with *Clostridium.*

The lysins of the invention may be used for preventing or reducing contamination of an object such as food with *Clostridium.* Contamination of an object with *Clostridium* means adhesion of viable *Clostridium* cells or spores thereof to the object. Reducing contamination with *Clostridium* means reducing the number of viable *Clostridium* cells adhering to the object. Determining contamination of objects with *Clostridium* is part of the general knowledge. For example, dilution plating of solutions or dispersions of homogenized food as done in the Examples or dilution plating of rinsing solution of other objects may be used, followed by counting bacterial colonies.

The composition of the invention may be a plant material or an extract thereof. Possible plant materials are as described above. An extract of plant material is an aqueous solution containing water-soluble proteins including the endolysin(s) of the invention that are present or expressed in said plant material. The extract preferably has water-insoluble components of the plant material removed. The extract may be produced from the plant material by homogenizing the plant material in water or an aqueous solution (e.g. as described in the following paragraph or the extraction buffer used in the experimental section) and, preferably, removing insoluble components from the plant material, e.g. by centrifugation or filtration. The product obtained is a crude protein extract and contains inter alia the lysin(s) active against *Clostridium.* Optionally, the extract may be purified as further described below to increase the content of the lysins used in the invention in the total protein content of the extract. The extract may be concentrated. Alternatively, the extract may be dried. After partial purification, the product may be considered a partially or semi-purified extract or concentrate.

The composition may be a solution that may be aqueous and that may, apart from the one or more lysins, contain a buffer. The buffer may be an inorganic or organic acid or salts thereof. An example of an inorganic acid is phosphoric acid or salts thereof. Examples of the organic acid are HEPES, acetic acid, succinic acid, tartaric acid, malic acid, benzoic acid, cinnamic acid, glycolic acid, lactic acid, citric acid, and ascorbic acid. Preferred organic acids are malic acid, lactic acid, citric acid, and ascorbic acid. The pH of the solution may generally be from 4 to 8, preferably from 5 to 8, more preferably from 5.5 to 7.5. If the object to which the composition is applied is meat, the pH of the solution may generally be from 4 to 8, preferably from 4.5 to 7, more preferably from 5.0 to 6.5, and even more preferably from 5.0 to 6.0. Further, the solution may contain isotonic agents such as glycerol or sodium chloride. A preferred salt to be used is sodium chloride. The aqueous solution containing said at least one endolysin may be a buffered aqueous solution that contains from 50 to 400 mM NaCl, preferably from 140 to 310 mM NaCl. The aqueous solution may further contain a sulfhydryl compound such as dithiothreitol (DTT), dithioerythritol, thioethanol or glutathione, preferably DTT. The concentration of the total of sulfhydryl compounds in the aqueous solution may be from 1 to 50 mM, preferably from 2 to 20 mM and more preferably from 4 to 10 mM. The concentration of the one or more lysins in the aqueous solution of the invention may be from 0.001 to 1 mg/ml, preferably from 0.01 to 0.5 mg/ml, and more preferably from 0.05 to 0.5 mg/ml. If more than one lysin active against *Clostridium* is employed, these concentrations relate to the total concentration of all lysins active against *Clostridium.*

Alternatively, the composition of the invention may be a solid such as a powder. Preferably, the composition is a lyophilized or dried powder containing said at least one lysin. The powder may contain additional components as mentioned above for the aqueous solution. The powder may be reconstituted, e.g. dissolved or suspended, in water or an aqueous solution before use.

The composition of the invention may contain at least 20, preferably at least 30, more preferably at least 50, even more preferably at least 75 % by weight of a lysin of the invention based on the total weight of protein in the composition. The content of lysin in the composition may be determined by subjecting the composition to SDS-PAGE and analyzing the obtained gel, after staining, by determining the intensity of bands on the gel. Thereby, intensity of bands due to lysin can be determined in relation to the intensity of bands due to all proteins in the composition. The total protein content in the composition may be determined using the well-known Biorad protein assay.

For preventing or reducing contamination of an object with *Clostridium,* the surface of the object may be wetted with a solution containing the one or more lysins of the invention, e.g. in concentrations as mentioned above. For example, the object such as food may be dipped into or sprayed with a solution of the one or more lysin of the invention. After wetting, the wetted object may be further processed or may be left to dry. In the case of food, the wetted food may be further processed such as by slicing or grinding and/or may be packed for shipping to customers or prepared for consumption.

For preventing or reducing contamination of food with *Clostridium,* the composition of the invention may be used as a food processing aid. For this purpose, the composition of the invention may be added to and/or mixed with food, followed by further processing of the food.

If the lysin is used for preventing or treating infection of an animal or human being with *Clostridium,* the lysin is administered to the animal or human. Examples of animals are farm animals such as poultry and cattle. Generally, a liquid or solid pharmaceutical composition containing the lysin is prepared for administration to the animal or human. Liquid compositions may be aqueous solutions as described above. Solid compositions may be powder containing the at least one lysin e.g. in freeze-dried form, or tablets obtained from such powder. Administration may be oral. In this case, the pharmaceutical preparation is one that allows passage through the stomach without being attacked by the acid medium in the stomach. The lysin should then be released from the pharmaceutical preparation in the intestine. Such pharmaceutical preparations are known in the art. Examples are tablets and capsules resistant to the acid medium in the stomach. It is further possible to administer orally a biological material such as *E. coli* or plant material containing expressed lysin to a patient. The lysin may be administered to a human adult in amounts of 1 mg to 1000 mg per day, preferably of from 10 mg to 250 mg per day to a human patient. Such amounts may also be administered to an animal.

In a probiotic approach, a patient may be treated by administering to the patient a genetically-modified microorganism expressing the at least one lysin. The genetically-modified microorganism may be a genetically-modified non-pathogenic *E. coli* or a lactic acid-producing microorganism as commonly employed in fermentation of milk products. Examples of lactic acid-producing microorganism are bacteria from the genera *Lactobacillus* such *Lactobacillus lactis* and *Bifidobacterium* such as *Bifidobacterium bifidum or Bifidobacterium breve.*

Another route of administration is by injection into the blood stream of a patient for preventing infection with *Clostridium.* For this purpose, the lysin may be dissolved in a physiological saline and the solution be sterilized.

### EXAMPLES

### MATERIALS AND METHODS

### Bacterial strains and growth conditions

*A. tumefaciens* strains used in this study are listed in Table 1. *E. coli* strain DH5α was used as a recipient for all cloning procedures. Both *E. coli* and *A. tumefaciens* cells were grown in LB medium at 37 °C or 30 °C, respectively. All media were supplemented, when necessary, with 100 µg/mL ampicillin, 50 µg/mL spectinomycin, 50 µg/mL kanamycin and 25 µg/mL rifampicin. *Clostridium perfringens* was grown in TSB (tryptic soy broth) medium at 37 °C in anaerobic conditions using AnaeroGen gas generating system (Oxoid). C. *perfringens* strains used in the study are listed in Table 2.

**Table 1. A. tumefaciens strains used in this invention**

| **Strains** | **Description** | **References** |
|---|---|---|
| ***A.tumefaciens*** | | |
| **GV3101** | (pMP90RK), nopaline, Rif^{r} | (Koncz and Shell 1986) |
| **GV3101(pICH20111)** | contains TMV virus-based 5' provector for cytosolic expression | Icon Genetics |
| **GV3101(pICH20030)** | contains TMV virus-based 5' provector for chloroplast targeting | |
| **GV3101(pICH1401)** | Contains integrase expression cassette | |
| **GV3101(pNMDV503)** | Contains TMV virus-based 3' provector with PlyCP26F | |
| **GV3101(pNMDV516)** | Contains TMV virus-based 3' provector with PlyCP39O | |
| **GV3101(pNMDV509)** | Contains TMV virus-based vector for cytosol expressed psm | |
| **GV3101(pNMDV600)** | Contains TMV virus-based vector for cytosol expressed ZP173 | |
| **GV3101(pNMDV599)** | Contains TMV virus-based vector for cytosol expressed CP25L | |
| **GV3101(pNMDV600)** | Contains TMV virus-based vector for cytosol expressed ZP278 | |
| **GV3101(pNMDV600)** | Contains TMV virus-based vector with chloroplast-targeted psm | |
| **GV3101(pNMDV600)** | Contains TMV virus-based vector with chloroplast-targeted ZP173 | |
| **GV3101(pNMDV600)** | Contains TMV virus-based vector with chloroplast-targeted ZP278 | |
| **GV3101(pNMDV602)** | Contains TMV virus-based vector chloroplast-targeted CP25L | |

**Table 2. C. perfringens strains used in this invention**

| **No.** | **Strain** | **Isolated from** | **Characteristics** | **Reference if possible** |
|---|---|---|---|---|
| 1 | NCTC8237 (ATCC 13124) | - | Type A, a-toxigenic, cpa and pfoA genes present. | Eastoe and Long, 1959 |
| | | | Type strain. | |
| 2 | NCTC8235 (ATCC 12922) | Stew | Type A, cpa and cpe genes present. | Hobbs BC, et al1953 |
| 3 | NCTC8239 (ATCC 12917) | Boiled salt beef | NCTC: Type A. a toxin gene positive. Contains a fragment of the enterotoxin gene. | Hobbs BC, et al. 1953. |
| | | | ATCC : Type D. Heat resistance of spores Agglutinating type 3. The presence of cpa, etx, and cpe genes was confirmed by PCR. | |
| 4 | NCTC8679 (ATCC 12920) | human, faeces, food poisoning | Type A. a toxin gene positive. | Hobbs BC, et al. 1953. |
| | | | Contains a fragment of the enterotoxin gene. | |
| 5 | NCTC9851 (ATCC 12925) | Braised heart | Type A. Agglutinating type 11 | Hobbs BC, et al. 1953. |
| | | | The presence of cpa and cpe genes was confirmed by PCR. | |
| 6 | NCTC11144 | Beef (food poisoning outbreak) | Type A. | Hobbs BC, et al. 1953. |
| 7 | NCTC8359 (ATCC 12915) | Stewed steak | Type A. The presence of cpa and cpe genes was confirmed by PCR. | Hobbs BC, et al. 1953. |
| 8 | NCTC8799 (ATCC 12924) | Roast meat | Type A. The presence of cpa and cpe genes was confirmed by PCR. | Hobbs BC, et al. 1953. |
| 9 | NCTC10613 | Minced beef, food poisoning | Type A. | - |
| 10 | NCTC8238 (ATCC 12916) | Boiled salt beef | Type A. The presence of cpa and cpe genes was confirmed by PCR. | Hobbs BC, et al, 1953. |
| 11 | NCTC8449 (ATCC 12921) | Steamed lamb | Type A. a-toxin gene positive. | Hobbs BC, et al. 1953. |
| | | | This isolate also contains a fragment of the enterotoxin gene | |
| 12 | NCTC8797 | Salt beef | Type A. a-toxin gene positive. | Hobbs BC, et al. 1953. |
| | | | This isolate also contains a fragment of the enterotoxin gene | |
| 13 | NCTC8798 | Meat rissole, food poisoning outbreak in a school | Type A. a-toxin gene positive. | Hobbs BC, et al. 1953. |
| | | | This isolate also contains a fragment of the enterotoxin gene | |
| 14 | NCTC10239 (ATCC 14809) | Rissoles | NCTC : Type A. a-toxin gene positive. This isolate also contains a fragment of the enterotoxin gene | Hobbs BC, et al. 1953. |
| | | | ATCC :Type D. The presence of cpa, etx, and cpe genes was confirmed by PCR. | |
| 15 | NCTC10240 (ATCC 1481) | Chicken | Type A. a-toxin gene positive. | Hobbs BC, et al., 1953. |
| | | | This isolate also contains a fragment of the enterotoxin gene | |
| 16 | NCTC8678 (ATCC 12919) | Human faeces, food poisoning | NCTC: Type A, Epsilon toxin - ve Agglutinating type 5 | Hobbs BC, et al. 1953. |
| | | | ATCC: Type D. The presence of cpa, etx, and cpe genes was confirmed by PCR. | |
| 17 | NCTC10614 | human, faeces, food poisoning case | Type A | Hobbs BC, et al. 1953. |
| 18 | NCTC10612 | human faeces(food poisoning outbreak) | Type A | Hobbs BC, et al. 1953. |
| 19 | NCTC10611 | - | Type A | Hobbs BC, et al. 1953. |
| 20 | NCTC8247 (ATCC 12918) | faeces | Type A. a-toxin gene positive. | Hobbs BC, et al. 1953. |
| | | | This isolate also contains a fragment of the enterotoxin gene | |
| 21 | NCTC10578 | - | Type A | - |
| 22 | NCTC2837 | - | Type A. a- toxin gene positive. | - |
| 23 | NCTC6785 (ATCC 10873) | - | Type A. a- toxin gene positive. | Epps, 1945 |
| 24 | DSM11778 | boulette (Hamburger) | Type A, betahaemolytic | - |
| 25 | DSM11779 | boulette (Hamburger) | Type A, betahaemolytic | - |
| 26 | DSM11780 | human faeces | Type A, betahaemolytic | - |
| 27 | DSM11781 | boulette (Hamburger) | Type A, betahaemolytic | - |
| 28 | DSM11782 | human faeces | Type A, betahaemolytic | - |
| 29 | DSM11783 | human faeces | Type A, non-haemolytic | - |
| 30 | DSM2943 (ATCC 25768) | - | Lechithinase-negative | Nakamura S, et al., 1976. |
| | | | The presence of the cpa gene was confirmed by PCR. | |
| 31 | DSM798 (ATCC 10543) | - | The presence of cpa, cpb2, and pfoA genes was confirmed by PCR. | Boyd MJ, et al. 1948. |

### Construction of expression vectors

Endolysins for expression in plants are described in Table 3. Plant-optimized gene coding sequences were synthetized by Eurofins, Austria (psm, PlyCP26F, PlyCP39O) and GenScript, USA (CP25L, ZP173, ZP278).

**Table 3. Endolysins, expressed in plants**

| Lysin (SEQ ID) | Origin | Characteristics | NCBI reference | Reference |
|---|---|---|---|---|
| psm (NO: 1) | *Clostridium* phage phiSM101 | GH25_Lyc-like and SH3b domain-containing protein | YP_699978.1 | Nariya et al. 2011 |
| CP25L (NO: 4) | *Clostridium* phage vB_CpeS-CP51 | N-acetylmuramoyl-L-alanine amidase | YP_00805894 8.1 | Gervasi et al. 2014 |
| PlyCP26F (NO: 5) | *Clostridium* phage phiCP26F | N-acetylmuramoyl-L-alanine amidase | YP_00700400 8.1 | Simmons et al. 2010 |
| PlyCP39O (NO: 6) | *Clostridium* phage phiCP39-O | N-acetylmuramoyl-L-alanine amidase | YP_00226543 5.1 | Simmons et al. 2010 |
| ZP173 (NO: 2) | *C. perfringens* CPE str.4969, prophage region | GH25_LytC-like and SH3b domain-containing protein | WP_0034693 59 | Schmitz et al. 2011 |
| ZP278 (NO: 3) | *C. perfringens* CPE str.4969, prophage region | GH25_Lyc-like domain-containing protein | WP_0034694 45.1 | Schmitz et al. 2011 |

The sequences were inserted as Bsal-Bsal fragments into the pICH31070 Δ lacZ plasmid (MagnICON deconstructed tobacco mosaic virus (TMV) system 3' provector (Marillonnet et al., 2004) and into the pICH29912 (for cytosolic expression) and pICH26201 (for expression in chloroplast) (assembled TMV-based MagnlCON vectors (Marillonnet et al., 2005)). Obtained plasmids were used to transform *A. tumefaciens* GV3101. *A. tumefaciens* strain containing pICH1401 with expression cassette of Streptomyces phage C31 integrase was used for co-infiltration with 5' and 3' provectors.

The T-DNA regions of obtained 3' provectors pNMDV503 (PlyCP26F) and pNMDV516 (PlyCP39O) and of assembled vectors pNMDV509 (cytosolic psm), pNMDV600 (cytosolic ZP173), pNMDV601 (cytosolic ZP278), pNMDV599 (cytosolic CP25L), pNMDV508 (chloroplast psm), pNMDV603 (chloroplast ZP_02640173), pNMDV604 (chloroplast ZP278), pNMDV602 (chloroplast CP25L) are presented in Figure 1. Restriction and modification enzymes from Thermo Scientific were used for all cloning steps.

### Example 1: Lysin expression in plants

*N. benthamiana* and *S*. *oleracea* plants were grown in a growth chamber at 25°C with a 16 h light and 8 h dark photoperiod. Four to six week - old plants were used for infiltration and for spraying with recombinant *A. tumefaciens.*

*A. tumefaciens* were grown overnight at 30°C in Luria-Bertani media containing 50 mg/l rifampicin and other appropriate antibiotics depending on the type of plasmids (50 mg/l kanamycin for selection of integrase, TMV 3' provector and TMV assembled vector; 50 mg/l carbenicilin for selection of 5' TMV provectors). *Agrobacterium* overnight cultures were adjusted to an OD₆₀₀ of 1.5, sedimented at 3220 × *g* for 5 min and resuspended in equal volume of tap water.

### Syringe infiltration of N. benthamiana leaves

Plant agroinfiltration was performed as previously described (Starkevič at al., 2015). A mix of three *A. tumefaciens* strains containing the 5' provector, 3' provector and integrase plasmid constructs was infiltrated into the abaxial side of the leaf using a syringe without a needle. MagnICON 5' provector modules for cytosol targeting (pICH20111) and chloroplast targeting (pICH20030) as well as the integrase expression vector pICH14011 were used together with 3'modules pNMDV503 and pNMDV516. Strains containing 5' and 3'provector modules were used at dilution 1:100, and the integrase cassette-containing strain at dilution 1:40. Plant leaves were observed and collected at five-six days post infiltration. pICH20111 (5' provector for cytosol expression), pICH20030 (5' provector for chloroplast expression) and plCH14011 (integrase) constructs are described in Kalthoff et al. (2010).

### Spraying of plant leaves

For lysin expression in *N. benthamiana* and in S. *oleracea* using assembled vectors, plant leaves were sprayed with a 1:1000 dilution of *A. tumefaciens* strain containing assembled vectors as described in Hahn et al. (2014). Spraying was performed with bacteria diluted in tap water containing 0.1% (v/v) Silwet L77 (Kurt Obermeier). Plant leaves were observed and collected at six-ten days post spraying.

### Example 2: Preparation of crude protein extracts for bacteriolytic activity tests

Frozen plant material was homogenized with chilled mortar and pestle and mixed with extraction buffer (50 mM NaH₂PO₄, 5 mM DTT, 150 mM NaCl, pH 7.5 at a *ratio* of 1 g of tissue to 5 ml of buffer. Cell debris were removed by centrifugation at 3220 g, at 4°C for 60 min. The supernatant was filtered and taken as total soluble protein.

### Purification of lysins

Purification of lysins followed the general scheme: homogenization of sample, clarification, HIC chromatography, followed by desalting and CEXC or AEXC column (Figure 2).

**ZP173.** Homogenized plant material extracted with buffer, containing 50 mM NaH₂PO₄, 2 mM DTT, 0.1% Tween 80, (pH 7.0), is clarified and loaded of butyl sepharose FF column. Colum is washed by 50 mM NaH₂PO₄, 2 mM DTT, 1.1 M (NH₄)₂SO₄, pH 7.0, 135-145 mS/cm. Lysin is eluted by step gradient at 30 % of elution buffer containing 50 mM NaH₂PO₄, 2 mM DTT, pH 7.0. After buffer exchange by concentration/dilution procedure (conductivity <7 ms/cm), the preparation is loaded on SP sepharose FF column. Column is washed by 50 mM NaH₂PO₄, 2 mM DTT, pH 5.0 and eluted by linear gradient (0-100 %) of 50 mM NaH₂PO₄, 2 mM DTT, 0.5 M NaCl, pH 5.0 (Figure 2A).

**ZP278.** Homogenized plant material extracted with buffer, containing 50 mM NaH₂PO₄, 5 mM DTT, 150 mM NaCl, (pH 7.5), is clarified and loaded of butyl sepharose FF column. Colum is washed by 50 mM NaH₂PO₄, 5 mM DTT, 1.2 M (NH₄)₂SO₄, pH 6.5, 145-155 mS/cm. Lysin is eluted by step gradient at 35 % of elution buffer containing 50 mM NaH₂PO₄, 5 mM DTT, pH 6.5. After buffer exchange by concentration/dilution procedure (conductivity <7 mS/cm), the preparation is loaded on DEAE sepharose FF column. Column is washed by 50 mM NaH₂PO₄, 5 mM DTT, pH 7.0 and eluted by linear gradient (0-100 %) of elution buffer (50 mM NaH₂PO₄, 5 mM DTT, 250 mM NaCl, pH 7.0 (5.5 - 6.5 mS/cm)) (Figure 2A).

**CP25L.** Homogenized plant material extracted with buffer, containing 50 mM NaH₂PO₄, 2 mM DTT, 100 mM NaCl, (pH 7.5), is clarified and loaded of butyl sepharose FF column. Colum is washed by 50 mM NaH₂PO₄, 2 mM DTT, 0.85 M (NH₄)₂SO₄, pH 6.5, 108-112 mS/cm. Lysin is eluted by step gradient at 20% of elution buffer containing 50 mM NaH₂PO₄, 2 mM DTT, pH 6.5. After buffer exchange by concentration/dilution procedure (conductivity <5 mS/cm), the preparation is loaded on Q sepharose FF column. Column is washed by 20 mM NaH₂PO₄, 2 mM DTT, pH 8.0 and eluted by linear gradient (0-25 %) of elution buffer (20 mM NaH₂PO₄, 2 mM DTT, 0.5 M NaCl, pH 8.0 (Figure 2A).

**PlyCP26F.** Homogenized plant material extracted with buffer, containing 50 mM NaH₂PO₄, 5 mM DTT, 150 mM NaCl, (pH 7.5), is clarified and loaded of butyl sepharose FF column. Colum is washed by 50 mM NaH₂PO₄, 5 mM DTT, 1.2 M (NH₄)₂SO₄, (pH 7.0), 145-150 mS/cm. Lysin is eluted by step gradient at 30% of elution buffer containing 50 mM NaH₂PO₄, 5 mM DTT, (pH 7.0). After buffer exchange by concentration/dilution procedure (conductivity < 8 mS/cm), the preparation is loaded on SP sepharose FF column. Column is washed by 50 mM NaH₂PO₄, 5 mM DTT, pH 6.0 (8-9 mS/cm) and eluted by step gradient at 20 % of elution buffer (50 mM NaH₂PO₄, 5 mM DTT, 1.0 M NaCl, pH 6.0) (Figure 2B).

**PlyCP39O.** Homogenized plant material extracted with buffer, containing 50 mM NaH₂PO₄, 5 mM DTT, 200 mM NaCl, (pH 7.5), is clarified and loaded of butyl sepharose FF column. Colum is washed by 50 mM NaH₂PO₄, 5 mM DTT, 1.2 M (NH₄)₂SO₄, pH 7.0, at 145-150 mS/cm. Lysin is eluted by step gradient at 35% of elution buffer containing 50 mM NaH₂PO₄, 5 mM DTT, pH 7.0. After buffer exchange by concentration/dilution procedure (conductivity < 16 mS/cm), the preparation iss loaded on SP sepharose FF column. Column is washed by 50 mM NaH₂PO₄, 5 mM DTT, 120 mM NaCl, pH 6.0 (15-16 mS/cm) and eluted by linear gradient (0-100 %) of elution buffer (50 mM NaH₂PO₄, 5 mM DTT, 0.5 M NaCl, pH 6.0) (Figure 2B).

**Psm.** Homogenized plant material extracted with buffer, containing 50 mM NaH₂PO₄, 150mM NaCl, (pH 5.0), is clarified and loaded of butyl sepharose FF column. Colum is washed by 50 mM NaH₂PO₄, 2 mM DTT, 1.2 M (NH₄)₂SO₄, pH 6.0, at 140-150 mS/cm. Lysin is eluted by step gradient at 35% of elution buffer containing 50 mM NaH₂PO₄, 2 mM DTT, pH 6.0. After buffer exchange by concentration/dilution procedure (conductivity < 6-7 mS/cm), the preparation is loaded on Q sepharose FF column. Column is washed by 50 mM NaH₂PO₄, 2 mM DTT pH 8.0 (6-7 mS/cm) and eluted by linear gradient (0-100 %) of elution buffer (50 mM NaH₂PO₄, 2 mM DTT, 250 mM NaCl, pH 8.0) (Figure 2B).

### Example 3: Bacteriolytic activity tests

### Turbidity reduction assay and cfu counts

Crude protein extracts or purified lysins were used in bacteriolytic activity tests. C. *perfringens* was grown in TSB under anaerobic conditions to OD₆₀₀appr. 0.7, sedimented by centrifugation and resuspended in a buffer of choice. 950 - 999 µL of bacterial suspension was mixed with 1-50 µL of purified lysin or crude protein extract and incubated at RT (room temperature). OD₆₀₀ measurements were taken with spectrophotometer (Genesys 20, Thermo Scientific) every 5-10 minutes. Experiments were performed in triplicate.

Serial dilutions for cfu count were done in 1xPBS pH 7.3 after 60 min. co-incubation of bacteria with plant extracts. 30 µL of each sample were plated on TSA plates: undiluted, diluted 10, 102, 103, 104 and 105 times. CFU counted following overnight incubation under anaerobic conditions at 37 °C.

For the determination whether a lysin derivative has at least 33 % of bacteriolytic activity as the parent lysin, the following procedure is employed: *C*. *perfringens* strain NCTC8237 is grown in TSB under anaerobic conditions to OD₆₀₀ appr. 0.7, sedimented by centrifugation and resuspended in 1xPBS pH 7.3 buffer. 950 - 999 µL of bacterial suspension is mixed with equal volumes of 1 - 50 µL of purified parent and derivative lysin of equal protein concentration. Serial dilutions for cfu count are done in 1xPBS pH 7.3 after 60 min. co-incubation of bacteria with lysin or its derivative. 30 µL of each sample are plated on TSA plates: undiluted, diluted 10, 10, 10³, 10⁴ and 10⁵ times with 1xPBS pH 7.3 buffer. Cfu are counted following overnight incubation under anaerobic conditions at 37 °C. If, at a suitable dilution, in the presence of the derivative lysin at most three times as many colonies are formed compared to the presence of the parent lysin, the derivative lysin is considered to have at least 33 % bacteriolytic activity of the parent lysin.

### Example 4: Bacteriolytic activity tests of crude lysin extracts in cooked meat

Crude protein extracts were prepared from agro-infiltrated *N. benthamiana* plants harvested at 6 dpi and using buffer containing 50 mM NaH₂PO₄, 300 mM NaCl, (pH 5.0). Protein extracts were diluted to conc. ~0.44-0.66 µg/µL. *C*. *perfringens* NCTC8237 strain was grown in TSB under anaerobic conditions to OD₆₀₀=0.24-0.26. Turkey breast meat and beef round meat was purchased from a local market. Chopped turkey meat was cooked in a pressure cooker for 30 min and chopped beef meat for 60 min.

100 µL of bacteria and 50 - 700 µL of protein extract or purified protein was added to 10 g of cooked meat and vortexed well. To equalize the volume of all samples, 1xPBS, pH 7.3 up to 1 mL was added to meat/bacteria sample. All the samples were placed into a 6 well multi-well plate and incubated under anaerobic conditions at room temperature.

Serial dilutions of analyzed samples were done in 1xPBS pH 7.3 and 30 µL of each sample plated on TSA plates: undiluted, diluted 10, 10², 10³, 10⁴, and 10⁵ times. Bacterial cfu/mL counting was done following overnight incubation under anaerobic conditions at 37 °C.

### RESULTS AND DISCUSSION

### Analysis of ZP173 and ZP278 sequences

Blast analysis of amino acid sequences of ZP_02640173 (WP_003469359) and ZP_02640278 (WP_003469445) (further in the text and in the figures named "ZP173" and "ZP278", respectively) revealed a GH25 domain in both proteins. Although both proteins are annotated in GenBank as bacterial cell wall hydrolases, close inspection of *Clostridium perfringens* F4969 genome (contigs NZ_ABDX01000023.1 and NZ_ABDX01000024.1) reveals the presence of several phage genes in close proximity of both ZP173 and ZP278. ZP173 is flanked by its right side with a gene coding for DNA adenine-specific methyltransferase, closely similar to that of *Clostridium* phage phiSM101. Also are located in proximity: the protein homologous to gp17 of *Clostridium* phage phiS63, the phage minor structural protein, the phage tail tape measure protein. ZP278 is also located in close proximity of phage-like proteins: phage minor structural protein, tape measure protein, capsid protein. Most likely, both ZP173 and ZP278 are localized in the remnants of prophage regions entrapped in C. *perfingens* F4969 genome and belongs to bacteriophage lytic enzymes family.

ZP173 sequence alignment with other described C. *perfringens* lysins demonstrated closest homologies to psm (and its close relative PlyCM), with highest percentage of identity at its carboxy-terminal end (177-335 aa, 64.6% of identity), while the N-terminal end (1-176 aa) is only 29.7% identical to the amino-terminal fragment of psm. ZP278 sequence alignment with other described C. *perfringens* lysins demonstrated closest homologies to psm (amino-terminal end, including the catalytic domain (1-179 aa, identity 47.2%), and to Ply3626 (C-terminal fragment (211-351 aa, 74.5% of identity)). Amino acid sequence alignments of ZP173 and ZP278 with its closest homologs PlyCM, psm and Ply3626 are presented in Figure 3.

### Construction of expression vectors

For the optimization of lysin expression in *Nicotiana benthamiana,* magnICON pro-vector system (Icon Genetics, Halle, Germany) previously described by Marillonet et al. (2004) and EP1385968, was used. A set of 5'TMV modules destined for cytosolic expression and chloroplast targeting (Figure 1 A) was used to test the expression levels of the recombinant lysins. The pro-vector modules (pNMDV503, and pNMDV516) were used for plant syringe-infiltration experiments. For plant spraying experiments assembled vectors (pNMDV509, pNMDV599, pNMDV600, pNMDV601, pNMDV508, pNMDV602, pNMDV603 and pNMDV604) were used (Figure 1 B and C).

### Lysin expression in plants

*N. benthamiana* leaves infiltrated with combinations of three *A. tumefaciens* strains (integrase, either of 5' provectors and either of 3'provectors) or sprayed with *A. tumefaciens* strains harboring assembled vectors were harvested five to ten days post infiltration and subjected to protein extraction. Leaf extracts with Laemmli buffer were analyzed by SDS-PAGE (Figure 4).

For cytosolic constructs, specific bands of expected size of about 38-39 kDa were detected in Coomassie-stained SDS-PAGE gels for psm and ZP173 lysins. Chloroplast-targeted psm migrated at the same level as cytosolic construct, showing complete cleavage of signal peptide. However, chloroplast targeted ZP173 migrated as a higher molecular weight protein, showing that chloroplast signal peptide was poorly cleaved. Theoretical ZP278 MW is 40.2 kDa, however, in SDS-PAGE, this lysin migrated as double band of about 2 kDa difference, the upper band corresponding to the theoretical size. The same tendency was observed for chloroplastic ZP278 construct (Figure 4A).

Cytosol-expressed PlyCP26F and PlyCP39O migrated as expected as 24.3 kDa proteins, but chloroplast constructs resulted in proteins of higher molecular weight, once again showing poor cleavage of chloroplast transit peptide. Both CP25L constructs, cytosolic and chloroplast, resulted in protein band of expected size of about 43 kDa. As cytosolic constructs in general performed better in *N. benthamiana* than chloroplast constructs, they were chosen for further experiments (Figure 4B).

Next, recombinant lysin expression levels in edible plants was determined. Young spinach plants were sprayed with *A. tumefaciens* strains carrying assembled lysin expression vectors. All three tested lysins (psm, ZP173 and ZP278) were expressed in spinach. Expression levels were superior using chloroplast constructs. ZP173 showed much better chloroplast transit peptide cleavage in spinach than in tobacco, but cytosolic ZP173 expression was very weak (Figure 4 C).

### Testing lysin activity against C. perfringens in vitro

Crude extracts of plant expressed C. *perfringens* phage lysins psm, ZP173, ZP278, CP25L, PlyCP26F and PlyCP39O were at first tested with C. *perfringens* type strain NCTC8237 (ATCC13124) (Figure 5). Incubation of bacterial suspension with plant-expressed extracts resulted in rapid OD reduction for ZP173, psm, ZP278 and CP25L (in order of reactivity). PlyCP26F and PlyCP39O were also able to clarify bacterial suspension, but acted more slowly and to a lesser extent (Figure 5A). CFU counts of bacterial suspension, treated with lysin extracts for one hour demonstrated high activity of all tested lysins. In this experiment, CP25L treatment reduced *C*. *perfringens* cfu/ml number by 5.7 log₁₀, ZP173 by 4.8 log₁₀, psm treatment reduced cfu/ml number by 4 log₁₀, PlyCP26F by 3.1 log₁₀ , ZP278 by 2.6 log₁₀, and PlyCP39O by 2 log₁₀ (Figure 5B).

We next tested in the similar set of experiments the extracts of spinach, agro-sprayed with ZP173 and ZP278 constructs. Despite lower relative levels of expression of both lysins in comparison with tobacco, spinach extracts of both proteins were active against all three tested *C. perfringens* strains (Figure 6). Extract of spinach sprayed with cytosol-expressed ZP173 construct decreased colony forming units count by 1.5-3.9 logarithmic units, depending on the *C. perfringens* strain. Chloroplast-targeted ZP173 decreased cfu/ml count by 1.8-3.6 log₁₀. Despite higher level of expression of both cytosol and chloroplast-targeted ZP278 in comparison with ZP173, the antimicrobial activity of this lysin was weaker for two tested strains than activity of ZP173 extracts.

### Lysins are active in salinity and acidity conditions commonly found in food

*Clostridium perfingens* is most commonly causes infections by ingestion of cooked meat products and catered food. For successful use of lysins as food additives, they should be active at pH and salinity conditions usually found in food. If lysins are to be used as food processing aids, they also should be able to exercise their activity in conditions used to prepare the food. We evaluated the influence of various factors on purified lysins activity: pH, concentration, temperature (Figure 7).

**NaCl.** Ready-to-eat meat products usually contain some amount of salt. In cured or cooked meat products (ham, sausages), NaCl concentration is as high as 150 - 350 mM. We tested influence of 50 mM to 500 mM NaCl concentration on activity of plant-produced lysins (Figure 7A). Activity of all three tested lysins, ZP173, ZP278 and CP25L, was positively influenced by increasing concentration of salt. As little as 50 mM of NaCl was sufficient to achieve almost maximum activity of ZP173, but activity of ZP278 and CP25L increased up to 500 mM of (highest concentration tested). These results demonstrate that salty food environment is perfectly suitable for the action of lysins. From other side, ZP173 demonstrates good activity even in buffer without NaCl and could be used for treatment of raw meat, which is low in sodium (NaCl concentration in raw meat and fish is in range of 10 mM, although it is several folds higher in some sea products).

pH. Most meat-based foods are slightly acidic in the pH range from 5.0 to 7.0, in part naturally, in part because of organic acids added to the processed product for better preservation. We tested lysin activity at pH range from 4.5 to 8.0. ZP173 activity was highest at pH 5.0 and decreased both towards lower and towards higher acidity, however, its activity remained high at pH range from 5.0 to 8.0 (Figure 7B). ZP278 behaved differently: its activity was the lowest at pH 5.5 and increased both towards lower and towards higher pH values. However, the differences of activity of this lysin at different pH values were not that significant and differed by no more than 1.5 log in cfu/ml count. CP25 reacted differently compared to both ZP lysins, as its activity was lowest at lowest tested pH value of 4.5 and constantly increased up to pH 8.0. The difference between lowest and highest activity of this lysin was about 2.5 logarithmic units of cfu/ml count. Considering the fact that both raw meat and processed meat products have a pH below 7.0, such conditions are optimal for ZP173, suitable for ZP278 and less optimal for CP25L which is most active at slightly alkaline medium.

### Lysins retain high activity after several days storage at ambient temperature

We looked for extreme temperature conditions in which lysins remain functional. ZP173 conserved its full activity after 30 min incubation at 37 °C to 44 °C. At 50 °C lysins activity declined and was almost completely abolished at 55 °C (Figure 8A).

We checked the influence of longer incubation times at ambient temperature and at 37 °C. ZP173 was stable at 37 °C for up to 3 days (Figure 8C) and conserved 50 % of activity after 6 weeks storage at room temperature (Figure 8B). CP25L was extremely stable at room temperature and after 6 weeks incubation, its activity remained practically unchanged. However, its activity started to decay after two days at 37 °C and after 7 days of incubation at 37 °C CP25L retained only about 20 % of its original activity (Figure 8B and C).

We did not detect any decline in activity of any of lysins incubated at 4 °C for several weeks (not shown). We conclude that purified lysins are extremely stable in refrigerated conditions and retain high activity after several days and weeks storage at ambient temperature.

The thermal stability of his-tagged E. coli-expressed CP25L had been evaluated by Gervasi et al. (2014). In the described study, CP25L was equaly stable at 4 °C, but began losing activity at room temperature after 15 days of storage; hence, plant-produced CP25L exhibits better stability than its bacterially produced counterpart. We conclude that purified ZP173 and CP25L lysins are extremely stable at 4 °C, retain high activity after several days and weeks of storage at ambient temperature, and exhibit significant functionality even after several days at 37 °C.

### Lysins are active against different C. perfringens food isolates.

Purified plant expressed lysins were further tested with different C. *perfringens* strains, isolated from food (Table 2). Cfu counting experiments of lysin-treated food isolates demonstrates different strain specificity to lysins, but all tested strains were more or less sensitive to all six lysins (Figure 9). Psm, ZP173 and ZP278 were most powerful in reducing cfu counts (from 1 to 8 Δlog₁₀), CP25L reduced cfu count from 1.5 to 4 Δlog₁₀ and PlyCP26F and PlyCP39O worked to a lesser extent (up to 1 Δlog₁₀). It should be pointed out that our experimental conditions (pH 5.5) are more optimal for ZP173 and ZP278 and maybe psm, than they are for CP25L which have better activity at more alkaline medium. It has also been published that the optimal pH conditions for PlyCP26F and PlyCP39O were 6.8 and 8.2, respectively (Simmons et al., 2010). Our choice of pH was related to acidity conditions most commonly found in food and is such conditions psm, ZP173 and ZP278 are most promising candidates, but for use with more alkaline foods the use of CP25L, PlyCP26F and PlyCP39O can be considered.

To better characterize the range of antibacterial activity, all six lysins were screened against a collection of 26 *C*. *perfringens* Type A strains isolated mostly from food or from human feces after documented cases of food poisoning. The results are shown in Fig. 12. All 26 strains were sensitive to treatment with one or more lysin(s) at the concentration used (10 µg/ml = 10 ppm). In general, treatment with psm, CP25L, ZP173 and ZP278 reduced cfu counts most dramatically, while PlyCP26F and PlyCP39O were less effective. Eradication of bacteria bellow the limit of detection was achieved for some strains by psm, ZP173 and ZP278 treatment (Fig. 12, marked by asterisks). Psm and ZP173 caused the most dramatic reduction in cfu counts, reaching a net difference of >4 logs for 7 and 8 strains, respectivelly. Four lysins (psm, CP25L, ZP173 and ZP278) were able to reduce cfu counts of 14 to 17 strains by 2 to 4 logs. For PlyCP26F and PlyCP39O, the maximum cfu reduction achieved was in the range of 0.5-2 logs. PlyCP26F could reduce cfu counts to that extent for 20 strains, and PlyCP39O for 6 strains only (Fig. 12).

No previous studies exist where the lytic activity of several lysins was compared using the same panel of C. perfringens food-relevant strains. Our results suggest that although some strains (NCTC8679, NCTC8798) appear more recalcitrant than others to treatment by all lysins tested, in general all strains were susceptible to lysin activity albeit with differences in strain-to strain susceptibility. Among the six tested lysins, only psm and ZP173 demonstrated very similar patterns of activity, most likely because of high homology of both proteins in their C-terminal cell wall binding domains. Our aim was to examine how *C*. *perfringens* could be eradicated under conditions similar to those found in food processing and/or preparation environments; thus, our justification for evaluating lysins at slightly acidic pH values found in many contamination-susceptible foods such as meat.

Based on these results, *C*. *perfringens* eradication is more successfully achieved by simultaneous treatment with several lysins (e.g. as a mixture) rather than by treatment with one individual lysin. For example, three plant-produced lysins (psm or ZP173, ZP278 and CP25L) could be used to control the broadest spectrum of *C*. *perfringens* food-relevant strains. In addition, as these lysins represent enzymes of different families, a synergistic effect of muramidases and alanine amidases could be achieved by using combinations of lysins.

### Testing lysin activity against C. perfringens in food

After demonstrating activity of plant psm, Z173 and ZP278 extracts *in vitro,* we checked if lysin extracts could be used to decrease the number of vegetative *C*. *perfringens* cells in food. To simulate conditions of improperly kept food, we used minced cooked turkey and beef meat at room temperature. 300 µg of psm-expressing *N. benthamiana* total protein extract was used to treat 10 g of meat, inoculated with *C*. *perfringens* at 10⁴ cfu/ml. In both types of meat after overnight incubation, cfu number was still unchanged in samples with psm, while in control samples bacteria cfu count grew by 4 logs and reached 10⁸ cfu/ml (Figure 10).

In another experiment we tested another lysin, purified ZP173. 20 µg or 50 µg of lysin was used to treat 10 g of minced cooked turkey meat. In this experiment, addition of lysin delayed *C*. *perfringens* outgrowth for 2h, but no significant difference in bacterial count was detected after ower night incubation (Figure 11).

Thus, crude protein extracts of plant-expressed *C*. *perfringens* lysins could be used to control the vegetative growth of *C*. *perfringens* bacteria in food, at least in some conditions.

Besides causing food poisoning, *C*. *perfringens* is etiologic agent of more serious diseases in humans: gas gangrene and enteritis necroticans. It is also causative agent of important poultry disease, necrotic enteritis. Altough necrotic enteritis is a multi-factorial disease and the predisposing factors that lead to the progresion to disease in poultry are still not completely understood, the feed or litter supplement of plant-expressed lysins could help to control the numbers of *Clostridia.*

Bacteriophage lysins are generaly regarded as safe and thus can be used for treatment of food and as human and animal therapeutic alternatives to antibiotics.

### Example 5: Bacteriolytic activity in cooked meat

### Method

*C. perfringens* NCTC8237 was used as a model pathogen strain. Cultures were grown to OD600~0.3 in TSB anaerobically, then diluted to OD600 =0.025. Samples of 10 g of minced cooked turkey meat were combined with 3 ml of citrate-phosphate buffer supplemented with 856 mM NaCl, pH 5.5 and 100 µL of diluted bacteria (4 log cfu/ml). 25 µg of purified lysin or 50 µg of nisin was added, thoroughly mixed and incubated at RT anaerobically. Nisin (Sigma) 5 mg/ml stock was prepared by dissolving powder in 0.02 N HCl. Serial dilutions of analysed samples were done in citrate-phosphate buffer supplemented with 256 mM NaCl, pH 5.5, and 50 µL of each sample were plated on TSA plates. Bacterial cfu/ml enumeration was done following overnight incubation under anaerobic conditions at 37 °C. A count of 25 cfu per plate of undiluted sample was considered the lower limit of detection (500 cfu/ml or 2.7 log10 cfu/ml) (Sutton (2011)).

### Results

We analyzed whether lysins are able to decrease the number of viable vegetative *C*. *perfringens* cells on food matrices. To simulate conditions of improperly kept food, we used minced cooked turkey at room temperature inoculated with vegetative *C*. *perfringens* cells at 104 cfu/ml. Currently, the polypeptide nisin is the only bacteriocin approved as a food preservative, which explains its increasingly common use by the food industry. Nisin has antimicrobial activity against Gram-positive bacteria and in particular against spore-forming species, and is able to inhibit both vegetative cells and the outgrowth of germinating spores (Gharsallaoui (2016)). However, although nisin showed inhibitory effect against spore outgrowth and vegetative cells of *C*. *perfringens* in laboratory conditions, no inhibitory effect of nisin was observed against *C*. *perfringens* spores inoculated in a meat model system (Udompijitkul (2012)), suggesting that nisin only arrests the outgrowth of germinated spores. Nisin action against vegetative cells can either be bactericidal or bacteriostatic depending on the concentration of nisin, concentration of bacteria, bacterial strain, physiological state and exposure conditions, and as reviewed by Garde (2014), in the majority of cases nisin's action is sporostatic rather than sporicidal.

In our experimental conditions, no bacteria following 5 µg/mg nisin treatment were detected after 2 hours, indicating that nisin is active against C. *perfringens* (Fig. 13). However, low titers of bacteria resurfaced after 18 hours and at 43 hours bacterial titers reached almost 5 log10 cfu. Thus, although nisin retarded multiplication of C. *perfringens* at the concentration used (which is nearly the maximal concentration allowed for its use as a food additive) it could not eradicate bacteria completely. In sharp contrast, two of the tested lysins, psm and ZP173, used at a two-fold lower (weight to weight) concentration than nisin (2.5 µg/ml), or a 10-20-times lower molar concentration, completely eradicated bacteria and no colonies were detected after 18 or 43 hours of incubation. CP25L was also very efficient and after 43 hours of incubation bacterial titers were reduced by 4 log₁₀ cfu compared to control (Fig. 13).

Thus, under the experimental conditions used, all three plant-expressed lysins efficiently protected cooked meat from C. *perfringens* spoilage, providing better protection than nisin. We conclude that plant-expressed lysins show utility as candidate food-protection antibacterials against C. *perfringens* when evaluated under conditions that realistically model food processing, preparation and serving scenarios.

Plant virus-based expression systems, in which the foreign mRNA encoding a protein of interest is amplified by the replicating virus, can produce very high levels of proteins in leaves and other tissues (Gleba and Giritch, (2011)). Furthermore, plant-based expression systems using Generally Recognized as Safe (GRAS) hosts (food species) have clear advantages when producing proteins for which extensive purification may need to be avoided. *C. perfringens* bacteriophage lysins produced in edible plants can be safely used as food additives or food processing aids even if only partially pure. Thus, despite possible expression of C. *perfringens* bacteriophage lysins in *E. coli,* plant- based expression is advantageous when the lysins are intended for food safety interventions.

### REFERENCES

Boyd, M. J., Logan, M. A, Tytell, A.A. A microbiological procedure for the assay of amino acids with Clostridium perfringens (welchii) BP6K. J. Biol. Chem. 1948. 174:1027-1035.
Eastoe, J.E.; Long,J.E. J. Appl. Bacteriol. The effect of nisin on the growth of cells and spores of Clostridium welchii. 1959. 22. 1-7.
Epps, H. M. R., Biochem. J., 39, 37 (1945).
Garde, S., Gómez-Torres, N., Hernández, M. & Ávila, M. Susceptibility of Clostridium perfringens to antimicrobials produced by lactic acid bacteria: Reuterin and nisin. Food Control 44, 22-25, doi:https://doi.org/10.1016/j.foodcont.2014.03.034 (2014). 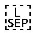
Gervasi, T., Horn, N., Wegmann, U., Dugo, G., Narbad, A., Mayer, M. J. Expression and delivery of an endolysin to combat Clostridium perfringens. Appl. Microbiol. Biotechnol. (2014) 98:2495-2505.
Gharsallaoui, A., Oulahal, N., Joly, C. & Degraeve, P. Nisin as a Food Preservative: Part 1: Physicochemical Properties, Antimicrobial Activity, and Main Uses. Crit Rev Food Sci Nutr 56, 1262-1274, doi:10.1080/10408398.2013.763765 (2016). 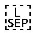
Gleba, Y.Y., Giritch, A. Plant Viral Vectors for Protein Expression. In Recent Advances in Plant Virology. Caister Academic Press. 2011 ISBN 978-1-904455-75-2; pp. 387-412.
Hahn, S., Giritch, A., Bartels, D., Bortesi, L., Gleba, Y., 2014. A novel and fully scalable Agrobacterium spray-based process for manufacturing cellulases and other cost-sensitive proteins in plants. Plant Biotechnol. 2015 Jun; 13 (5):708-16.
Hobbs, B.C, Smith M.E, Oakley,C.L, Warrack, G.H and Cruickshank, JC. Clostridium welchii food poisoning. J. Hyg. (Lond). 51(1): 75-101, 1953.
Kalthoff, D., Giritch, A., Geisler, K., Bettman, U., Klimyuk, V., Hehnen, H.R., Gleba, Y., Beer, M. Immunization with plant-expressed hemagglutinin protects chickens from lethal highly pathogenic avian influenza virus H5N1 challenge infection. J. Virol. 2010 84, 12002-12010
Koncz, C. and Shell, J. 1986. The promoter of TL-DNA gene 5 controls the tissue-specific expression of chimaeric genes carried by a novel type of Agrobacterium binary vector. Mol. Gen. Genet. 204:383-396
Li, J., McClane, B. A. Further Comparison of Temperature Effects on Growth and Survival of Clostridium perfringens Type A Isolates Carrying a Chromosomal or Plasmid-Borne Enterotoxin Gene. Appl Environ Microbiol. 2006 Jul; 72(7): 4561-4568.
Lindström, M., Heikinheimo, A., Lahti, P., Korkeala H., Novel insights into the epidemiology of Clostridium perfringens type A food poisoning. Food Microbiology 28 (2011) 192e198.
Mangen, M-J. J., Bouwknegt, M., Friesema, I. H. M., Haagsma, J. A., Kortbeek, L. M., Tariq, L., Wilson, M, van Pelt W., Havelaar A. H. Cost-of-illness and disease burden of food-related pathogens in the Netherlands, 2011. International Journal of Food Microbiology 196 (2015) 84-93.
Marillonnet, S., Giritch, A., Gils, M., Kandzia, R., Klimyuk, V., Gleba, Y., 2004. In planta engineering of viral RNA replicons: Efficient assembly by recombination of DNA modules delivered by Agrobacterium. Proc. Natl. Acad. Sci. U.S.A. 101 (18), 6852-6857.
Marillonnet, S., Thoeringer, C., Kandzia, R., Klimyuk, V., Gleba, Y., 2005. Systemic Agrobacterium tumefaciens-mediated transfection of viral replicons for efficient transient expression in plants. Nat. Biotechnol. 23(6), 718-723.
Miller, R. W., E. J. Skinner, A. Sulakvelidze, G. F. Mathis, and C. L. Hofacre. Bacteriophage therapy for control of necrotic enteritis of broiler chickens experimentally infected with Clostridium perfringens. Avian Dis. 2010, 54(1):33-40.
S Nakamura, S, Okada, I, Mizuno, J, Nishida, S. Lecithinase-negative variants of Clostridium perfringens; the identity of C. plagarum with C. perfringens. Can. J. Microbiol. 22: 1497-1501, 1976.
Nariya, H., Miyata, Sh., Tamai E., Sekiya, H., Maki, J., Okabe, A. Identification and characterization of a putative endolysin encoded by episomal phage phiSM101 of Clostridium perfringens. Appl Microbiol Biotechnol (2011) 90:1973-1979.
Schmitz, J. E., Maria Cristina Ossiprandi, M. C., Rumah, K. R., Fischetti, V. A. Lytic enzyme discovery through multigenomic sequence analysis in Clostridium perfringens. Appl Microbiol Biotechnol (2011) 89:1783-1795.
Schulz, S., Stephan, A., Hahn, S., Bortesi, L., Jarczowski, F., Bettmann, U., Paschke, A. K., Tusé, D., Stahl, C. H., Giritch, A., Gleba, Y. Broad and efficient control of major foodborne pathogenic strains of Escherichia coli by mixtures of plant-produced colicins. Proc Natl Acad Sci U S A. 2015 Oct 6;112(40): E5454-60.
Simmons, M., Donovan, D. M., Siragusa G. R., S. Seal, B. S. Recombinant Expression of Two Bacteriophage Proteins That Lyse Clostridium perfringens and Share Identical Sequences in the C-Terminal Cell Wall Binding Domain of the Molecules but Are Dissimilar in Their N-Terminal Active Domains. J Agric Food Chem. 2010 October 13; 58(19): 10330-10337.
Starkevič, U., Bortesi, L., Virgailis, M., Ružauskas, M., Giritch, A., Račanskienė, A. High-yield production of a functional bacteriophage lysin with antipneumococcal activity using a plant virus-based expression system. J Biotechnol. 2015 Apr 20; 200:10-6.
Sutton, S. Accuracy of Plate Count. Vol. 17 (2011).
Udompijitkul, P., Paredes-Sabja, D. & Sarker, M. R. Inhibitory effects of nisin against Clostridium perfringens food poisoning and nonfood-borne isolates. J Food Sci 77, M51-56, doi:10.1111/j.1750-3841.2011.02475.x (2012).
Xiao,Y., Wagendorp, A., Moezelaar, R., Abee, T., H. J. Wells-Bennika M. H. J. A Wide Variety of Clostridium perfringens Type A Food-Borne Isolates That Carry a Chromosomal cpe Gene Belong to One Multilocus Sequence Typing Cluster. Applied and Environmental Microbiology 2012 October Vol.78 (19), 7060-7068.
Zimmer, M., Vukov, N., Scherer, S., Loessner, M. J. The murein hydrolase of the bacteriophage phi3626 dual lysis system is active against all tested Clostridium perfringens strains. Appl Environ Microbiol. 2002 Nov; 68(11):5311-7.

## Claims

1. A method of preventing or reducing contamination of food with *Clostridium,* comprising contacting said food with a composition comprising at least one endolysin active against said *Clostridium,*
wherein at least one of said at least one endolysin is ZP173 comprising the amino acid sequence of SEQ ID NO: 2 or a derivative of this endolysin, and
wherein said derivative or endolysin has a sequence identity of at least 90% to the amino acid sequence of SEQ ID NO: 2; or said derivative or endolysin has from 1 to 30 amino acid substitutions, insertions and/or deletions compared to the amino acid sequence of SEQ ID NO: 2.

2. The method according to claim 1, wherein said composition is or comprises a plant material or extract thereof, wherein the plant material is a material from a plant having expressed said at least one endolysin, preferably an edible plant having expressed said at least one endolysin.

3. The method according to any one of claims 1 or 2, wherein said composition is an aqueous solution containing said at least one endolysin; or said composition is a lyophilized or dried powder containing said at least one endolysin, said powder may be dissolved or suspended in water or an aqueous solution before use.

4. The method according to claim 3, wherein said aqueous solution containing said at least one endolysin is a buffered aqueous solution that contains from 50 to 400 mM NaCl, preferably from 140 to 310 mM, and may further contain a sulfhydryl compound.

5. The method according to any one of claims 1 to 4, wherein said food is sprayed with an aqueous solution containing said at least one endolysin or is immersed into an aqueous solution containing said at least one endolysin.

6. The method according to any one of claims 1 to 5, wherein said food is meat, preferably said meat is raw meat or cooked meat; or said food is gravy.

7. The method according to any one of claims 1 to 6, wherein said *Clostridium* is *Clostridium perfringens,* preferably said *Clostridium perfringens* is anyone or more selected from of Type A, Type B, Type C, Type D and Type **E,** preferably a Type A strain containing enterotoxin CPE.

8. The method according to claim 1, wherein said derivative or endolysin has a sequence identity of at least 95%, preferably at least 97%, to the amino acid sequence of SEQ ID NO: 2; or
said derivative or endolysin has from 1 to 15, preferably from 1 to 10, amino acid substitutions, insertions and/or deletions compared to the amino acid sequence of SEQ ID NO: 2.

9. The method according to claim 1, wherein said composition is a buffered aqueous solution that contains from 150 to 700 mM NaCl, preferably from 200 to 550 mM NaCl, and/or the composition is an aqueous solution of a pH from 4 to 8, preferably from 4.5 to 7, more preferably from 5.0 to 6.5, and even more preferably from 5.0 to 6.0.

10. The method according to claim 2, wherein said plant material is material from a plant selected from the group consisting of spinach, chard, beetroot, carrot, sugar beet, *Nicotiana tabacum, Nicotiana benthamiana* and/or said plant material is one or more leaves, roots, tubers, or seeds, or a crushed, milled or comminuted product of said leaves, roots, tubers, or seeds.

11. A process of producing a composition comprising at least one endolysin active against *Clostridium,* said process comprising the following steps:
(i) expressing said at least one endolysin in a plant, preferably an edible plant,
(ii) harvesting plant material containing expressed endolysin from said plant,
(iii) extracting said at least one endolysin from said plant material using an aqueous buffer to obtain a composition containing said at least one endolysin,
(iv) optionally removing undesired contaminants from said composition,
wherein at least one of said at least one endolysin is ZP173 comprising the amino acid sequence of SEQ ID NO: 2 or a derivative of this endolysin,
wherein said derivative or endolysin has a sequence identity of at least 90% to the amino acid sequence of SEQ ID NO: 2; or said derivative or endolysin has from 1 to 30 amino acid substitutions, insertions and/or deletions compared to the amino acid sequence of SEQ ID NO: 2.

12. The process according to claim 11, wherein step (iv) comprises, in this order, a hydrophobic interaction chromatography step, a desalting step, and an ion exchange chromatography step.

13. A composition comprising an endolysin active against *Clostridium,* wherein said endolysin is ZP173 comprising the amino acid sequence of SEQ ID NO: 2 or a derivative of this endolysin,
wherein said derivative or endolysin has a sequence identity of at least 80% to the amino acid sequence of SEQ ID NO: 2; or said derivative or endolysin has from 1 to 30 amino acid substitutions, insertions and/or deletions compared to the amino acid sequence of SEQ ID NO: 2.

14. The composition according to claim 13 for use in a method of treating or preventing infection with *Clostridium* such as *Clostridium difficile* or *Clostridium perfringens.*

## Patentansprüche

1. Verfahren zum Verhindern oder Verringern der Kontamination von Lebensmitteln mit *Clostridium,* umfassend das Inkontaktbringen des Lebensmittels mit einer Zusammensetzung, die mindestens ein gegen das *Clostridium* aktives Endolysin umfasst,
wobei das mindestens eine Endolysin mindestens eines der mindestens einen Endolysine ZP173 ist, das die Aminosäuresequenz von SEQ ID NO: 2 oder ein Derivat dieses Endolysins umfasst, und
wobei das Derivat oder das Endolysin eine Sequenzidentität von mindestens 90 % zu der Aminosäuresequenz von SEQ ID NO: 2 aufweist; oder das Derivat oder Endolysin 1 bis 30 Aminosäuresubstitutionen, -insertionen und/oder -deletionen im Vergleich zu der Aminosäuresequenz von SEQ **ID** NO: 2 aufweist.

2. Das Verfahren nach Anspruch 1, wobei die Zusammensetzung ein Pflanzenmaterial oder einen Extrakt davon ist oder umfasst, wobei das Pflanzenmaterial ein Material aus einer Pflanze ist, die das mindestens eine Endolysin exprimiert hat, vorzugsweise eine essbare Pflanze, die das mindestens eine Endolysin exprimiert hat.

3. Das Verfahren nach einem der Ansprüche 1 oder 2, wobei die Zusammensetzung eine wässrige Lösung ist, die das mindestens eine Endolysin enthält; oder die Zusammensetzung ein lyophilisiertes oder getrocknetes Pulver ist, das das mindestens eine Endolysin enthält, wobei das Pulver vor der Verwendung in Wasser oder einer wässrigen Lösung gelöst oder suspendiert werden kann.

4. Das Verfahren nach Anspruch 3, wobei die wässrige Lösung, die das mindestens eine Endolysin enthält, eine gepufferte wässrige Lösung ist, die 50 bis 400 mM NaCl, vorzugsweise 140 bis 310 mM, enthält und zusätzlich eine Sulfhydrylverbindung enthalten kann.

5. Das Verfahren nach einem der Ansprüche 1 bis 4, wobei das Lebensmittel mit einer wässrigen Lösung besprüht wird, die das mindestens eine Endolysin enthält, oder in eine wässrige Lösung getaucht wird, die das mindestens eine Endolysin enthält.

6. Das Verfahren nach einem der Ansprüche 1 bis 5, wobei das Lebensmittel Fleisch ist, wobei das Fleisch vorzugsweise rohes Fleisch oder gekochtes Fleisch ist; oder das Lebensmittel eine Soße ist.

7. Das Verfahren nach einem der Ansprüche 1 bis 6, wobei das *Clostridium Clostridium perfringens* ist, vorzugsweise wobei das *Clostridium perfringens* eines oder mehrere aus Typ A, Typ B, Typ C, Typ D und Typ E ausgewählt ist, vorzugsweise ein Typ-A-Stamm, der Enterotoxin CPE enthält.

8. Das Verfahren nach Anspruch 1, wobei das Derivat oder Endolysin eine Sequenzidentität von mindestens 95 %, vorzugsweise mindestens 97 %, mit der Aminosäuresequenz von SEQ ID NO: 2 aufweist; oder
das Derivat oder Endolysin im Vergleich zur Aminosäuresequenz von SEQ ID NO: 2 1 bis 15, vorzugsweise 1 bis 10 Aminosäuresubstitutionen, -insertionen und/oder - deletionen aufweist.

9. Das Verfahren nach Anspruch 1, wobei die Zusammensetzung eine gepufferte wässrige Lösung ist, die 150 bis 700 mM NaCl, vorzugsweise 200 bis 550 mM NaCl, enthält und/oder die Zusammensetzung eine wässrige Lösung mit einem pH-Wert von 4 bis 8, vorzugsweise von 4,5 bis 7, noch bevorzugter von 5,0 bis 6,5 und noch bevorzugter von 5,0 bis 6,0 ist.

10. Das Verfahren nach Anspruch 2, wobei das Pflanzenmaterial Material aus einer Pflanze ist, die aus der Gruppe ausgewählt ist, die aus Spinat, Mangold, Rote Beete, Karotten, Zuckerrüben, *Nicotiana tabacum, Nicotiana benthamiana* besteht, und/oder das Pflanzenmaterial ein oder mehrere Blätter, Wurzeln, Knollen oder Samen oder ein zerkleinertes, gemahlenes oder zermahlenes Produkt dieser Blätter, Wurzeln, Knollen oder Samen ist.

11. Verfahren zur Herstellung einer Zusammensetzung, die mindestens ein gegen *Clostridium* aktives Endolysin umfasst, wobei das Verfahren die folgenden Schritte umfasst:
(i) Exprimieren des mindestens einen Endolysins in einer Pflanze, vorzugsweise einer essbaren Pflanze,
(ii) Ernten von Pflanzenmaterial, das das exprimierte Endolysin enthält, aus der Pflanze,
(iii) Extrahieren des mindestens einen Endolysins aus dem Pflanzenmaterial unter Verwendung eines wässrigen Puffers, um eine Zusammensetzung zu erhalten, die das mindestens eine Endolysin enthält,
(iv) gegebenenfalls Entfernen unerwünschter Verunreinigungen aus der Zusammensetzung,
wobei mindestens eines des mindestens einen Endolysins ZP173 ist, das die Aminosäuresequenz von SEQ ID NO: 2 umfasst, oder ein Derivat dieses Endolysins,
wobei das Derivat oder Endolysin eine Sequenzidentität von mindestens 90 % zur Aminosäuresequenz von SEQ ID NO: 2 aufweist; oder das Derivat oder Endolysin im Vergleich zur Aminosäuresequenz von SEQ ID NO:2 1 bis 30 Aminosäuresubstitutionen, -insertionen und/oder -deletionen aufweist.

12. Das Verfahren nach Anspruch 11, wobei Schritt (iv) in dieser Reihenfolge einen hydrophoben Interaktionschromatographieschritt, einen Entsalzungsschritt und einen Ionenaustauschchromatographieschritt umfasst.

13. Zusammensetzung, die ein gegen *Clostridium* aktives Endolysin umfasst, wobei das Endolysin ZP173 ist, das die Aminosäuresequenz von SEQ ID NO: 2 umfasst, oder ein Derivat dieses Endolysins,
wobei das Derivat oder Endolysin eine Sequenzidentität von mindestens 80 % zu der Aminosäuresequenz von SEQ **ID** NO: 2 aufweist; oder das Derivat oder Endolysin 1 bis 30 Aminosäuresubstitutionen, -insertionen und/oder -deletionen im Vergleich zu der Aminosäuresequenz von SEQ **ID** NO: 2 aufweist.

14. Die Zusammensetzung gemäß Anspruch 13 zur Verwendung in einem Verfahren zur Behandlung oder Vorbeugung einer Infektion mit *Clostridium,* wie *Clostridium difficile* oder *Clostridium perfringens.*

## Revendications

1. Méthode de prévention ou de réduction de la contamination d'un aliment par *Clostridium,* comprenant le contact dudit aliment avec une composition comprenant au moins une endolysine active contre ledit *Clostridium,*
dans laquelle au moins une de ladite au moins une endolysine est ZP173 comprenant la séquence d'acides aminés de SEQ ID NO : 2 ou un dérivé de cette endolysine, et
dans laquelle ledit dérivé ou ladite endolysine présente une identité de séquence d'au moins 90 % par rapport à la séquence d'acides aminés de SEQ ID NO : 2 ; ou ledit dérivé ou ladite endolysine présente de 1 à 30 substitutions, insertions et/ou délétions d'acides aminés par rapport à la séquence d'acides aminés de SEQ ID NO : 2.

2. Méthode selon la revendication 1, dans laquelle ladite composition est ou comprend une matière végétale ou un extrait de celle-ci, dans laquelle la matière végétale est une matière provenant d'une plante ayant exprimé ladite au moins une endolysine, de préférence une plante comestible ayant exprimé ladite au moins une endolysine.

3. Méthode selon l'une quelconque des revendications 1 ou 2, dans laquelle ladite composition est une solution aqueuse contenant ladite au moins une endolysine ; ou ladite composition est une poudre lyophilisée ou séchée contenant ladite au moins une endolysine, ladite poudre peut être dissoute ou mise en suspension dans de l'eau ou une solution aqueuse avant utilisation.

4. Méthode selon la revendication 3, dans laquelle ladite solution aqueuse contenant ladite au moins une endolysine est une solution aqueuse tamponnée qui contient de 50 à 400 mM de NaCl, de préférence de 140 à 310 mM, et peut en outre contenir un composé sulfhydryle.

5. Méthode selon l'une quelconque des revendications 1 à 4, dans laquelle ledit aliment est pulvérisé avec une solution aqueuse contenant ladite au moins une endolysine ou est immergé dans une solution aqueuse contenant ladite au moins une endolysine.

6. Méthode selon l'une quelconque des revendications 1 à 5, dans laquelle ledit aliment est de la viande, de préférence ladite viande est de la viande crue ou de la viande cuite ; ou ledit aliment est de la sauce.

7. Méthode selon l'une quelconque des revendications 1 à 6, dans laquelle ledit *Clostridium* est *Clostridium perfringens,* de préférence ledit *Clostridium perfringens* est un ou plusieurs éléments quelconques sélectionnés parmi type A, type B, type C, type D et type E, de préférence une souche de type A contenant l'entérotoxine CPE.

8. Méthode selon la revendication 1, dans laquelle ledit dérivé ou ladite endolysine présente une identité de séquence d'au moins 95 %, de préférence d'au moins 97 %, par rapport à la séquence d'acides aminés de SEQ ID NO : 2 ; ou
ledit dérivé ou ladite endolysine présente de 1 à 15, de préférence de 1 à 10, substitutions, insertions et/ou délétions d'acides aminés par rapport à la séquence d'acides aminés de SEQ ID NO : 2.

9. Méthode selon la revendication 1, dans laquelle ladite composition est une solution aqueuse tamponnée qui contient de 150 à 700 mM de NaCl, de préférence de 200 à 550 mM de NaCl, et/ou la composition est une solution aqueuse d'un pH de 4 à 8, de préférence de 4,5 à 7, plus préférentiellement de 5,0 à 6,5, et encore plus préférentiellement de 5,0 à 6,0.

10. Méthode selon la revendication 2, dans laquelle ladite matière végétale est une matière provenant d'une plante sélectionnée dans le groupe consistant en les épinards, la bette à cardes, la betterave, la carotte, la betterave sucrière, *Nicotiana tabacum, Nicotiana benthamiana* et/ou ladite matière végétale est un(e) ou plusieurs feuilles, racines, tubercules ou graines, ou un produit écrasé, moulu ou comminuté desdit(e)s feuilles, racines, tubercules ou graines.

11. Procédé de production d'une composition comprenant au moins une endolysine active contre *Clostridium,* ledit procédé comprenant les étapes suivantes :
(i) l'expression de ladite au moins une endolysine dans une plante, de préférence une plante comestible,
(ii) la récolte de matière végétale contenant de l'endolysine exprimée à partir de ladite plante,
(iii) l'extraction de ladite au moins une endolysine à partir de ladite matière végétale à l'aide d'un tampon aqueux pour obtenir une composition contenant ladite au moins une endolysine,
(iv) l'élimination optionnelle de contaminants indésirables de ladite composition, dans lequel au moins une de ladite au moins une endolysine est ZP173 comprenant la séquence d'acides aminés de SEQ ID NO : 2 ou un dérivé de cette endolysine, dans lequel ledit dérivé ou ladite endolysine présente une identité de séquence d'au moins 90 % par rapport à la séquence d'acides aminés de SEQ ID NO : 2 ; ou ledit dérivé ou ladite endolysine présente de 1 à 30 substitutions, insertions et/ou délétions d'acides aminés par rapport à la séquence d'acides aminés de SEQ ID NO : 2.

12. Procédé selon la revendication 11, dans lequel l'étape (iv) comprend, dans cet ordre, une étape de chromatographie d'interaction hydrophobe, une étape de dessalage et une étape de chromatographie d'échange d'ions.

13. Composition comprenant une endolysine active contre *Clostridium,* dans laquelle ladite endolysine est ZP173 comprenant la séquence d'acides aminés de SEQ ID NO : 2 ou un dérivé de cette endolysine,
dans laquelle ledit dérivé ou ladite endolysine présente une identité de séquence d'au moins 80 % par rapport à la séquence d'acides aminés de SEQ ID NO : 2 ; ou ledit dérivé ou ladite endolysine présente de 1 à 30 substitutions, insertions et/ou délétions d'acides aminés par rapport à la séquence d'acides aminés de SEQ ID NO : 2.

14. Composition selon la revendication 13 pour une utilisation dans une méthode de traitement ou de prévention d'une infection par *Clostridium,* tel que *Clostridium* difficile ou *Clostridium perfringens.*
